# EUROPEAN PATENT SPECIFICATION

(11) **EP 1 206 494 B1**
(45) Date of publication and mention of the grant of the patent: **03.11.2004**
(21) Application number: 00949767.8
(22) Date of filing: 02.08.2000
(51) Int. Cl.: C07K 16/28, C07K 7/08, C07K 1/107, G06F 17/50

(54) **COMPUTATIONAL DESIGN METHODS FOR MAKING MOLECULAR MIMETICS**
BERECHNUNGSMETHODEN ZUR HERSTELLUNG VON MOLEKULAREN MIMETIKA
METHODES DE CONCEPTION PAR ORDINATEUR DESTINEES A LA PRODUCTION DE MIMETIQUES MOLECULAIRES

(30) Priority: 02.08.1999 EP 99401968
(43) Date of publication of application: 22.05.2002
(73) Proprietor: SYNT:EM S.A., 30000 Nîmes (FR)
(72) Inventor: CASSET, Florence, c/o Synt:em, 30000 Nîmes (FR); GRANIER, Claude, oImmunoanlyse et Innov en Biol Cl, 34060 Montpellier Cedex 02 (FR); KACZOREK, Michel, c/o Synt:em, 30000 Nîmes (FR); LAHANA, Roger, c/o Synt:em, F-30000 Nîmes (FR); REES, Anthony, c/o Synt:em, 30000 Nîmes (FR); ROUX, Florence, c/o Synt:em, 30000 Nîmes (FR)
(74) Representative: Brasnett, Adrian Hugh
(86) International application number: PCT/GB2000/002972
(87) International publication number: WO 2001/009191

(56) References cited:
- WO-A-92/09625
- WO-A-93/24518
- US-A- 5 834 250
- US-A- 5 846 933
- GRASSY GERARD ET AL: "COMPUTER-ASSISTED RATIONAL DESIGN OF IMMUNOSUPPRESSIVE COMPOUNDS." NATURE BIOTECHNOLOGY, vol. 16, no. 8, August 1998 (1998-08), pages 748-752, XP002152660 ISSN: 1087-0156
- TUCHSCHERER G ET AL: "EXTENDING THE CONCEPT OF TEMPLATE-ASSEMBLED SYNTHETIC PROTEINS." JOURNAL OF PEPTIDE RESEARCH, vol. 54, no. 3, 1999, pages 185-194, XP002152662 ISSN: 1397-002X
- PELUSO S ET AL: "Protein Mimetics (TASP) by Sequential Condensation of Peptide Loops to an Immobilised Topological Template" TETRAHEDRON,NL,ELSEVIER SCIENCE PUBLISHERS, AMSTERDAM, vol. 53, no. 21, 26 May 1997 (1997-05-26), pages 7231-7236, XP004105702 ISSN: 0040-4020
- MURALI RAMACHANDRAN ET AL: "Structure-based design of immunologically active therapeutic peptides." IMMUNOLOGIC RESEARCH, vol. 17, no. 1-2, January 1998 (1998-01), pages 163-169, XP000960672 ISSN: 0257-277X
- FAVRE ET AL: "STRUCTURAL MIMICRY OF CANONICAL CONFORMATIONS IN ANTIBODY HYPERVARIABLE LOOPS USING CYCLIC PEPTIDES CONTAINING A HETEROCHIRAL DIPROLINE TEMPLATE" JOURNAL OF THE AMERICAN CHEMICAL SOCIETY,XX,XX, vol. 121, 31 March 1999 (1999-03-31), pages 2679-2685, XP002137023 ISSN: 0002-7863
- PRIDE MICHAEL W ET AL: "Molecular mimicry of hepatitis B surface antigen by an antiidiotype-derived synthetic peptide." PROCEEDINGS OF THE NATIONAL ACADEMY OF SCIENCES OF THE UNITED STATES, vol. 89, no. 24, 1992, pages 11900-11904, XP002152663 1992 ISSN: 0027-8424
- LEVI MICHAEL ET AL: "A COMPLEMENTARITY-DETERMINING REGION SYNTHETIC PEPTIDE ACTS AS A MINIANTIBODY AND NEUTRALIZES HUMAN IMMUNODEFICIENCY VIRUS TYPE 1 IN VITRO." PROCEEDINGS OF THE NATIONAL ACADEMY OF SCIENCES OF THE UNITED STATES, vol. 90, no. 10, 1993, pages 4374-4378, XP002152664 1993 ISSN: 0027-8424
- VITA C ET AL: "NOVEL MINIPROTEINS ENGINEERED BY THE TRANSFER OF ACTIVE SITES TO SMALL NATURAL SCAFFOLDS" BIOPOLYMERS,US,NEW YORK, NY, vol. 47, 1998, pages 93-100, XP002924847 ISSN: 0006-3525

## Description

### Field of the invention.

The present invention relates to the design and synthesis of a molecule which mimics the activity of a parent molecule. Such a molecule is generally referred to as a mimetic. For example, the parent molecule may be a protein which is known to interact with a certain "target" molecule, and the invention may relate to generating a mimetic which is different from the parent molecule but which also interacts with the target molecule.

More particularly, the invention relates to methods of designing a mimetic. Also described are methods of identifying active portions of a parent molecule for incorporation in a mimetic, a method of screening mimetics for the desired activity, apparatus for performing the methods, uses of the mimetic, and pharmaceutical compositions comprising the mimetic.

### Background of the invention.

Many of the reactions which are studied in biochemistry may be stated as the interaction between a protein and a second, "target" molecule (which is frequently itself a protein). Such interactions include antigen-antibody, enzyme-substrate and hormone-receptor interactions. In many of these reactions the portion of the protein which is responsible for the interaction represents only a small proportion of the total protein molecule, and this portion is conventionally referred to as the "active region" of the protein.

The design of small oligopeptide, or peptide-like mimetics to reproduce the activity of large natural proteins based on mimicking the active region has numerous applications in both therapeutics and diagnostics. Where the activity region of interest is located within a continuous sequence of the protein, methods have been described in which the conformational requirements of such regions can be met by various chemical cross-linking or other synthetic strategies. However, where the activity region(s) of a protein consist of discontinuous segments of the polypeptide chain, the problem of mimetic design is particularly acute.

A number of researchers have attempted to design a molecule which mimics a known protein, in the sense that the mimetic too is capable of interacting with the target molecule. In constructing a mimetic, one first identifies the active region(s) of the known protein (for example in the case of an antibody-antigen interaction one identifies which region(s) of the antibody enable binding to the antigen), and then searches for a mimetic which emulates the active region. Since the active region of the known protein is relatively small, it is hoped that a mimetic will be found which is much smaller (e.g. in molecular weight) than the protein, and correspondingly easier and cheaper to synthesise. Such a mimetic could be used as a convenient substitute for the protein, as an agent for interacting with the target molecule.

Reineke et al. ("A synthetic mimic of a discontinuous binding site on interleukin-10", Nature Biotechnology, Vol. 17, 1999, p271-275) proposed designing a mimic molecule which mimics a binding site of the interleukin-10 protein by the following steps. To begin with, a large library of short peptides were synthesised, each of which corresponded to a short section of interleukin 10. The binding of each of these peptides to the target (in this case an antibody against interleukin-10) was then tested individually by an assay technique, to identify potentially relevant peptides.

The next step in the mimicking procedure was to synthesise further peptides based on the potentially relevant peptides. That is, Reineke et al. selected one of the potentially relevant peptides derived earlier, and synthesised all possible peptides wherein each amino acid of the selected peptide was replaced by one of the other 19 L-amino acid (i.e. the amino acids were replaced in all possible ways by every other possible amino acid). The binding activity of each of the resultant "replacement peptides" was tested. This indicated which of the amino acids were important for activity of the protein.

Some of the amino acid replacements led to enhanced activity of the replacement peptide compared with the corresponding peptide upon which the replacement peptide was were based. Further peptides were produced in which combinations of these "enhancing replacements" were incorporated. Peptides, having the best binding to the target were selected for use as mimetics.

Reineke et al. described how this process could be carried even further. Namely, they tried joining together two of the mimetics identified by the above method, to create a new combined mimetic.

Specifically, they proposed joining two of the derived peptide strands by a linker peptide. The length of the linker sequence was chosen to keep the distance between the two mimicking peptides the same as in the known protein. The distance in the known protein was based on a previously determined crystallographic structure.

Amino acid replacements were also made in this combined peptide. As before, this was done by the systematic replacement of amino acids. Thus an optimised peptide was produced based on the combined peptide with "enhancing replacements".

Reineke et al. also produced cyclic molecules by the introduction of two cysteine residues within the optimised peptide to allow the formation of a disulphide bridge. In fact, they systematically synthesised every possible such cyclic molecule (i.e. they tried out every possible way of replacing two residues of the optimised peptide with two cysteine residues). This resulted in a huge library of cyclised peptides, which were then individually screened to determine their ability to bind to the target. Again, this was a highly labour intensive operation.

In similar work, Falcioni et al. ("Peptidomimetic compounds that inhibit antigen presentation by autoimmune disease- associated class II major histocompatibility molecules", Nature Biotechnology Vol. 17, 1999, p 562-567) used peptide libraries to identify a heptapeptide with high affinity for rheumatoid arthritis-associated class II major histocompatibility (MHC) molecules. A number of substitutions were then made in the lead heptapeptide and the binding affinities of the resultant peptides tested to find mimetics of the lead heptapeptide which had comparable or enhanced binding affinities. The chosen mimetics were then tested for their ability to inhibit T-cell responses to processed protein antigens presented by the MHC molecules.

Laune et al., (Systematic Exploration of the Antigen Binding Activity of Synthetic Peptides Isolated from the Variable Regions of Immunoglobulins, The Journal of the Biochemistry Society, Vol 272, 1997, 30937-30944) describe "the alanine scanning method" for the identification of important amino acids. In this approach a library of peptides was synthesised, each peptide corresponding to a short section of the active region of a known protein and the activity tested by a binding assay. Peptide sequences which bind the target were identified in this way.

Next, hexapeptides were synthesised which corresponded to the identified peptide binding sequences, further hexapeptides based on these binding hexapeptides were then synthesised. Each further hexapeptide differed from the corresponding binding hexapeptide by the replacement of a single amino acid by another amino acid, in this case alanine. The activity of each of the further hexapeptides was then measured. Whenever the replacement of an amino acid by alanine led to a drastic reduction in the activity of the peptide, that amino acid was identified as being important. Laune et al also went on to identify the location of the important amino acids on the 3D X-Ray crystallographic structure.

In view of the very high number of syntheses required, and the requirement that the binding ability of each synthesised peptide be measured, the above procedures are extremely labour intensive. It is therefore clearly of great importance to utilise the information gained as a result of such procedures in a rational and efficient manner.

A related approach is the "TASP method" (Tuchscherer, "Template Assembled Synthetic Proteins: Condensation of a Multifunctional Peptide to Topological Template via Chemioselective Ligation" , Tetrahedron Letters, 1993, Vol 34, No 52, pp.8419-8422; Tuscherer et al ., "The Tasp Concept: Mimetics of Peptide Ligands, Protein Surfaces and Folding units", Tetrahedron, 1993, Vol. 49, no.17, pp.3559-3575; Tuchscherer et al., "Protein design: Or the Threshold of Functional Properties", 1998, Vol. 47, pp. 63-73). Tuscherer et al. ("The Tasp Concept: Mimetics of Peptide Ligands, Protein Surfaces and Folding units") helical peptides from the MHC molecule HLA-A2 were fused to a peptidic template base to mimic the folding topology of HLA-A2. Further alterations were made to the helices including (i)the mutation of residues not thought to be involved in binding to the receptor to residues favouring helix formation and (ii) mutations to improve amphiphilicity. The structures were tested for helix formation using circular dichroism. Antibodies raised to the TASP molecule were specific for the native MHC molecule.

In the TASP approach individual structural motifs are tethered by their ends to an external scaffold, though the structural context of each motif, one to another , is not necessarily maintained. Mutations are made in the mimic molecules to improve the topology and the effect of these mutations tested by structural determination or by biological activity. This again is a labourious process. Moreover, the TASP molecules are technically difficult to produce.

Grassy et al. (Nature Biotechnology 1998 v.16 pp.748-752) generate an in silico library of 279,936 peptides derived from the heavy chain of HLA class I. The peptides are filtered using static and dynamic filters to provide five lead compounds which are synthesised and tested *in vivo*.

### Summary of the invention

The present invention aims to provide methods which are of use in designing a molecule which mimics the activity of a parent molecule.

The present invention further aims to employ molecular dynamics computer simulation to rationalise the quest for mimetics.

Once a set of potentially active chemical groups (e.g. amino acid residues) in a parent molecule has been determined experimentally (e.g. by the alanine scanning method experimentally identifying active chemical groups within active (peptide) fragments of a parent molecule such as an antibody), this set of chemical groups is screened computationally to predict whether or not, in the parent molecule, they are accessible. A chemical group which is accessible is more likely to be associated with an activity of the parent molecule. Those chemical groups which are predicted to be accessible may then be incorporated into a mimetic. The invention employs a level of structural understanding to short-cut the trial-and-error identification of active chemical groups described above.

Specifically, the invention provides a method of designing a mimetic which exhibits an activity associated with a parent molecule, the method including:
identifying a plurality of chemical groups of the parent molecule which are associated with activity;
generating a mimetic including the chemical groups associated with activity and a structure which links the chemical groups;
computationally performing a molecular dynamics simulation of the generated mimetic;
determining from the simulation whether the mimetic obeys a predetermined criterion; and
varying the linking structure to produce a second mimetic which better obeys the predetermined criterion.

The invention provides in a further aspect a method of designing a mimetic of an antibody binding region, the method comprising:
providing the three-dimensional structure of at least the target binding region of the antibody variable domain;
identifying amino acid residues within the region potentially associated with target binding based on experimental data;
computationally determining the respective degree of accessibility of each of the identified amino acid residues within the three-dimensional structure;
selecting a plurality of amino acid residues associated with target-binding activity from among the identified amino acids;
generating a mimetic including the plurality of amino acids, the amino acids groups having within the mimetic a relative geometrical arrangement corresponding to their determined relative geometrical arrangement in the parent antibody,
computationally performing a molecular dynamics simulation of the generated mimetic;
determining from the simulation whether the mimetic obeys a predetermined criterion; and
varying the linking structure to produce a second mimetic which better obeys the predetermined criterion.

A method of identifying, within a parent molecule which is known to have a predetermined activity, at least one chemical group of the parent molecule which is associated with that activity may comprise the steps of:
computationally determining the three-dimensional structure of at least a portion of the parent molecule, the portion including an active region of the parent molecule;
selecting a plurality of chemical groups within the active region potentially associated with the activity based on experimental data;
computationally determining the respective degree of accessibility of each of the selected chemical groups within the three-dimensional structure; and
identifying at least one chemical group associated with activity from among the selected chemical groups based on their respective degree of accessibility.

In the statements of methods according to the invention the order of the steps is not a limitation on the scope of the invention. For example, the 3D model can be generated after the chemical groups are selected.

The experimental selection of potentially active chemical groups may include the steps of: selecting a plurality of sections of the active region of the parent molecule, each section being a string of chemical groups; determining experimentally an activity associated with each string; for each string, repeatedly selecting a chemical group within it and modifying that chemical group (e.g. removing it or replacing it, for example by chemical or genetic methods) and determining the change in activity caused by this modification; and selecting chemical groups from within the string on the basis of the change in activity.

The degree of accessibility of a particular chemical group may be a measure of the exposure of that chemical group in the parent molecule, either to a solvent molecule (such as water with or without ions) or to a target molecule to which the parent molecule binds.

The method may include a further step of designing, or optionally chemically synthesising, a molecule including the identified chemical groups(s) for acting as a mimetic.

A mimetic can be designed by the present invention to take into account the folding structure of the parent molecule (i.e. how the active region of the molecule is folded in three dimensions). If the parent molecule includes a loop on which is located at least one first active chemical group, and at least one second active chemical group which is not located on the loop, the mimetic is designed to include a cyclic portion which includes the first chemical group (s), and outside the cyclic portion the second chemical group (s).

Preferably, in the method of designing a mimetic of an antibody binding region, the amino acid residues which are associated with target binding include at least one first residue which is located on a first loop of the antibody variable domain, and at least a second residue which is not located on the first loop of the antibody variable domain; and a mimetic is designed which comprises a first cyclic portion including the first residue, and outside the first cyclic portion the second residue.

Preferably, at least some of the inactive part of the mimetic (i. e. at least some of the mimetic other than the active chemical groups) comprises one or more chemical groups ("framework chemical groups") which are present in the parent molecule proximal to the active chemical groups but which are not themselves known to be active. For example, the framework chemical groups may be chemical groups which are not thought to be potentially active (for example, they were not identified as being active during the experimental selection process) or thought not to be accessible to the target in the parent molecule, or a combination of these.

If the active chemical groups are located in the parent molecule such that there are one or more active chemical groups on each of a first loop and a second loop of the parent molecule, then the mimetic may be designed to include two cyclic portions, each of which includes the active chemical groups of the respective loops. Preferably, the linking structure within the mimetic between the cyclic portions is by structural elements which maintain the relative orientation of the cyclic portions to resemble the relative orientation of the loops within the parent molecule.

For example, if at least one second residue is located on a second loop of the antibody variable domain, the mimetic may comprise a first cyclic portion including the residue, and outside the first cyclic portion a second cyclic portion comprising at least said second residue

This scheme may be extended to cases in which the active chemical groups to be included in the mimetic include chemical group(s) located on three, four or even more loops of the parent molecule. The mimetic may contain a number of cyclic portions up to this number of loops, each cyclic portion including the respective active group(s).

For example, the methods of the invention may be performed two (or more) times, on each occasion designing a first molecule which has at least one cyclic portion containing active group(s) and further active group(s) outside the cyclic portion. Then a second molecule may be derived, based on the two (or more) first molecules and containing substantially all the structure of the two first molecules. For example, in the case that the parent molecule is an antibody, one first molecule might be based on the active groups of the CDR3 of the heavy chain and CDR 3 of the light chain, while another first molecule might be based on the active groups of the CDR2 of the heavy chain and the CDR 2 of the light chain. The two first molecules could be joined to form a mimetic which contains structure to emulate both the CDR2 and CDR3 of the heavy and light chains.

The method may comprise a further step of chemically synthesising a molecule designed by the method of the invention.

Whereas the mimetic may emulate (if not necessarily replicates) in its topology the folding structure of the parent molecule, an alternative is to concentrate on aiming to produce a mimetic in which the active chemical groups have the relative geometrical relationship which they have in the parent molecule, even if the structure which connects them is topologically dissimilar to that of the parent molecule.

A mimetic may be designed in which the geometrical relationship between active chemical groups corresponds to the geometrical relationship between the corresponding chemical groups in the parent molecule as seen from the point of view of the target molecule, e.g. as viewed from a face of the parent molecule with which the target molecule interacts.

A method of designing a mimetic which exhibits an activity associated with a parent molecule may comprise:
identifying a plurality of chemical groups of the parent molecule associated with activity;
determining the relative geometrical relationship between the chemical groups within the parent molecule as viewed from one direction;
generating a mimetic including the plurality of chemical groups, the chemical groups having within the mimetic a relative geometrical arrangement corresponding to their determined relative geometrical arrangement in the parent molecule.

Beside the active chemical groups, the mimetic comprises a linking structure made up of chemical linkage groups which link the active groups so as to essentially match the geometry of those active chemical groups in the known molecule. Such chemical linkage groups can be chemical groups of the same type as those of which the parent molecule is comprised. They may even be sections of the parent molecule. For example if the parent molecule is a protein the chemical linkage groups could be amino acids but a linking structure of non-peptidic nature could also be used.

Preferably the chemical linkage groups are chosen with reference to the three-dimensional structure of the parent molecule or portion thereof. It is preferred that the linking structure is designed so that the geometrical arrangement of the active groups mimics that of these groups in the parent molecule when viewed from one direction. The active groups of the parent molecule have a certain geometrical arrangement as viewed from one direction. When viewing a parent molecule from one direction, the active groups may be considered as lying in a section according to that view.

The linkage groups are preferably selected to maintain the arrangement of the active groups within this section. There may be further chemical groups or atoms, i.e. other than the active groups, which are found within this section in the parent molecule. In this situation, it is preferred that the linking structure is designed so that these further groups or atoms are retained within the linking structure, and that the arrangement of the atoms in that section is maintained. In this way, a mimetic may be designed in which active groups and further chemical groups lying in the section are linked together. Groups (or the backbone moieties to which said groups are attached) which are not contiguous in the parent molecule are brought together in a contiguous structure by the linking structure.

For example, where the parent molecule is a protein, there may be amino acid side chains which project into the same section as the active amino acids. The mimetic is preferably designed so that these side chains are present in the mimetic. In this way amino acid side chains which are not neighbouring in primary sequence may be brought together in the mimetic. Preferably the linking structure is selected so that these side chains have the same orientation in the mimetic as in the parent. The linking structure may be peptidic or non-peptidic, in order to provide appropriate orientation of said side chains.

The invention proposes in general terms that a mimetic is selected to include a plurality of active chemical groups joined by a linking structure and that molecular dynamics simulation is employed to select the linking structure.

As for the step of varying the first mimetic to produce the second mimetic, in the case that the parent molecule is a protein for example, the linking structure may be varied by the addition to, or replacement of, the amino acids which are involved in the linkage in the first mimetic by one or more amino acids. For example, amino acid groups of different chemical nature could be used, for example hydrophilic groups could be replaced with hydrophobic groups such as valine or leucine. Proline could be used to provide "kinks" which are characteristically produced by the introduction of proline into peptides. In this way further mimetics may be designed in which the active groups are linked by various chemical linkage groups. The variation in chemical linkage groups may result in a variation in the geometry of the chemical. linkage groups and hence the geometry of the mimetic. Such variation may stabilise the conformation of the mimetic, e.g. in a form resembling the folding structure of the parent molecule. For example, by introducing chemical linkage groups which are present in the parent molecule the stability or conformation of the mimetic may be improved.

Optionally, the second mimetic may be itself varied to generate a third mimetic which satisfies the same (or a different) criterion to a greater degree than the second mimetic. In other words, it would be possible to make this process iterative.

Although in the method of the invention the molecular simulation is used to produce an improved second mimetic (i.e. as part of the design process), the screening of potential mimetics on the basis of a criterion based on molecular dynamics modelling has a wider applicability. For example, it can be used following a step of designing of a plurality of potential mimetics, as a way of determining which of the set of molecules are worth synthesising.

Molecular dynamics is used in modelling to screen potential mimetics which are "virtual molecules" (i.e. which do not necessarily have physical existence but which exist as representations within a computer or stored on a recording medium), to identify which of the potential mimetics are worthy of synthesis.

The likelihood of a mimetic exhibiting the activity of a parent molecule is evaluated by performing a molecular dynamics simulation of all or part of the mimetic (as discussed further below), and evaluating it by a criterion based on the molecular dynamics simulation. A molecule which is evaluated to be likely to exhibit the activity may then be synthesised and its activity tested experimentally, in vitro and/or in vivo.

The evaluation of a mimetic may include performing individual molecular dynamics simulations on parts of the mimetic. In this way, a mimetic which is synthesised may result from a series of evaluations of molecular dynamics simulations of parts of the mimetic. For example, in the case where the mimetic includes one or more loops, the mimetic may be evaluated by molecular dynamics simulations of each individual loop.

Following the design of a mimetic, the mimetic can be synthesised by methods known to the person skilled in the art. Such methods are discussed elsewhere herein.

It is preferred that the mimetics have an arrangement of active chemical groups which is similar to the arrangement of those active chemical groups in the parent molecule.

A mimetic provided by the invention may be formulated into a pharmaceutical composition.

The applicability of the invention will be described below with reference to a pharmaceutical application, but the invention is applicable in principle to other chemical fields, for example the design of pesticides or other agrochemicals.

### Description of the Drawings.

Figure 1 shows a sequence alignment of ST40. The CDRs loop are underlined. The important (bold) and less important (bold, italic) amino acids residues are represented. The residues selected to be included in the mimetic are boxed.
Figure 2 is a ribbon representation of the 3D structure of ST40 and localization of the important (black) and less important (dark grey) amino acid residues determined by alanine Scanning. The cut-off for residues with or without accessibility to the antigen is indicated by the dotted line. Above the line is the binding surface of the CDRs, and below the line is the surface interacting with the constant domain of the antibody. The regions where active amino acids will be selected and excluded are thus defined as being above and below the line respectively.
Figure 3 shows a ribbon representation of the CDRs of ST40 (from the top). The amino acids selected to be included in the mimetic are shown in black.
Figure 4 shows a space filling representation of the mimetic 3D structure with a Lys extended by a 3-mercaptopropionic acid (3MP) group. The structure is the average structure over the 100 last ps or a molecular dynamics of 300ps (before mutation). The amino acid lie has been mutated to a Lys extended by 3-MP which is represented in black.
Figure 5 shows (A) a sensorgram of CD4 and irrelevant proteins (8A6, 9E8, TnC) when the mimetic is immobilized on the sensor chip; and (B) a sensorgram of binding competition by injection of pre-incubated CD4 + ST40 and CD4 + 9E8 when the mimetic is immobilized on the sensor chip.
Figure 6 shows the fragments of ST40 used (grey) for the dynamics simulation in water.
Figure 7 is a representation of H3, L1 and L3 CDRs during the MD simulation of ST40 when all amino acids are fixed except H3, L1 and L3 (Black: starting conforner, Grey: conformer at 50, 100, 150, 200, 250 and 300 ps of the MD simulation).
Figure 8 is a representation of H3, L1 and L3 CDRs during the MD simulation of ST40 with ions (Black; starting conformer, Grey: conformer at 50, 100, 150, 200, 250 and 300 ps).
Figure 9 shows a PCA representation (cp1, cp2) of the two MD simulations of ST40. The autocorrelogram was calculated only for the backbone of the amino acids allowed to move during the MD (Grey without ion, black: with ions).
Figure 10 is a representation of the H3 mimetics R and E. The starting conformations are represented in grey and the conformations after 100 ps in black.
Figure 11 is a representation of the H3L1 mimetic RM. The starting conformation is represented in grey and the conformation after 100 ps of MD in black.
Figure 12 is a representation of the H3L1 mimetic EC, and an MD simulation thereof. The starting conformation is represented in grey and the conformation after 100 ps of MD simulation in black.
Figure 13 is a representation of Cα atoms of EC2. THE starting conformation is represented in black and the conformations at 50, 80, 100, 120 and 150 ps in grey.
Figure 14 shows the PCA representation (cp1, cp2) of the MD simulation of ST40 (black) and EC2 (grey).
Figure 15 shows a representation of the H3L1L3 mimetic E[WFK]2
Figure 16 is a representation of the H3L1L3 mimetics a)E[WFK]2 and b) E[WFK]3. The starting conformations are represented in black and the conformations after 300ps in grey.
Figure 17 shows a PCA representation (cp1, cp2) of the MD simulation of ST40 (Black) E[WFK]2 (dark grey) and E[WFK]3 (light grey) .

### Detailed Description of the Invention.

### Molecule

The term "molecule" is used herein to include chemical compounds of high molecular weight (even up to "macro-molecules"), including proteins. For very large proteins, e.g. Factor XIII (molecular weight about 300 kDa) or large proteins, e.g. antibodies (molecular weights 100-200 kDa), a small proportion of the protein may be modelled where appropriate. For example, the variable region of an antibody may be modelled, in this case the model would be of a region of about 50-400 amino acids. For a medium sized protein, e.g. lysozyme (molecular weight 15 kDa) the model may be based on the whole structure, but could be based on part of the structure, e.g. active site. Small molecules may also be modelled, for example epitopes could be modelled(small regions of antigens which are known to bind antibodies) have sizes ranging from 5 to 10 amino acids.

### Three dimensional model.

In this application the term 3D model is understood to mean a three dimensional structure represented within a computer. The representation may be derived from first principles using molecular modelling techniques, or alternatively may incorporate any available from crystallographic data or nuclear magnetic resonance data or structural predictions based on homology databases.

### Folding structure.

The folding structure of a molecule refers to the three dimensional arrangement of the chemical groups of that molecule. Where the molecule is a protein, the folding structure includes secondary structure, tertiary structure and quaternary structure of the peptide chains. Similarly RNA folding structures are a result of either RNA secondary structure e.g T-RNA-like structures which are involved in replication of some RNA viruses, or from tertiary structures e.g. pseudoknots, or a combination of these.

### Generating a mimetic.

Generating a mimetic refers to both generation in terms of physical synthesis of the molecule and generating a virtual molecule by computer.

### Amino acids.

Amino acids may be any naturally occurring or non-naturally occurring amino acids, including acidic, basic, hydrophobic, hydrophilic, polar, large, small or exotic α-amino acids and any other amine acid compounds. This includes D-amino acids and amino acid derivatives are also included, such as alkylated (e.g. methylated or acetylated), hydroxylated, phosphorylated, or glycosylated amino acids.

### Parent molecule.

A parent molecule is a molecule to be mimicked. The parent molecule has a function which is to be mimicked and the mimetic will display this function. Usually the parent molecule will display an activity to be mimicked and may be known as an "active starting molecule". Typically, parent molecules interact with a second molecule called the "target molecule" and the activity or function of the parent molecule may be the binding to the target, or a downstream effect of this binding, e.g a biological activity. However, the invention is not limited the situation where the parent molecule interacts with a target.

The invention is particularly applicable to oligomeric or polymeric molecules which have an inherent three dimensional structure.

The parent molecule may be a protein, for example an antibody, but the term "parent molecule" is not limited in this respect (as discussed below) and includes also other protein molecules (including glycoproteins), e.g. enzymes, receptors, antigens and hormones. Other bioactive molecules which could be mimicked include carbohydrates and nucleic acids (e.g. the mimicking of RNA secondary and or tertiary structures). The invention includes mimicking the active surfaces of inorganic molecules, such as inorganic polymeric molecules or the active sites of catalysts.

The parent molecules of the invention may be a naturally occurring molecule, e.g. naturally occurring antibodies, or parts of molecules. They may also be synthetic parent molecules, e.g. synthetic antibody fragments (see below). The invention may be used to mimetic peptides from peptide display libraries, e.g. peptides which have been selected from a phage display library on the basis of their binding to a particular target, in this situation it would be possible to mimic a peptide having a particular binding activity as chosen from the library.

The parent molecules are made up of a number of chemical groups, e.g. in a protein the constituent chemical groups are amino acids, in a nucleic acid the constituents are nucleotides.

### Binding molecules.

The invertion is particularly relevant to molecules which bind a target (binding molecules). In this way, mimetics of the binding molecule could bind the target, for example if the target was a cell surface receptor, mimetics could be made to antibodies against the cell surface receptor and these mimetics could block the receptor.

Mimetics can be made to either the parent molecule or its target, in other words, a mimetic can be made to either member of a binding interaction. Mimetics could be made to either the antibody or the antigen in an antigen:antibody interaction. Therefore antigenic epitopes could be mimicked, these would include the looped epitopes commonly found on viral surfaces, e.g. certain epitopes of rhinoviruses, human immunodeficiency virus and polioviruses.

### Members of the immunoglobulin superfamily

The present invention is suitable for the generation of mimetics of the members of the immunoglobulin superfamily, including antibodies, T-cell receptors, MHC molecules (HLA in humans) and other cell surface receptors such as N-CAM particularly antibody molecules. The invention is particularly relevant to mimicking of antibodies.

The term "antibody" should be construed as covering any binding substance having a binding domain with the required specificity. Thus the parent molecules of the invention include antibody fragments, derivatives, functional equivalents and homologues of antibodies, including synthetic molecules and molecules whose shape mimics that of an antibody enabling it to bind an antigen or epitope.

Example antibody fragments, capable of binding an antigen or other binding partner are the Fab fragment consisting of the VL, VH, Cl and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

### Cell surface receptors.

As mentioned above the invention includes mimetics of cell surface receptors of the immunoglobulin superfamily, e.g. T-cell receptors, and N-CAM. The invention also includes other cell surface receptors, e.g. the members of the integrin family of cell surface receptors.

### Other parent molecules.

The parent molecule may be any molecule for which a 3D model of at least part of the molecule exists or can be produced. For example the parent molecule may be an enzyme. This includes proteases such as the serine proteases (e.g. chymotrypsin), polymerases, replicases, telomerases, transcriptases, ribonucleases and many other enzymes which are known to those skilled in the art. Other possible parent molecules include, insulin, tumour necrosis factor, tissue plasminogen activating factor.

### Target molecule.

The target molecule is a molecule which interacts with the parent molecule, by interaction with the active chemical groups of the active region of that parent molecule. Where the molecule is a catalyst the target could be the reactant or the product or the intermediate reactant-product complex. Where the molecule is an enzyme, the target could be the substrate, or the substrate-product intermediates or the product. Where the molecule is an antibody the target would be the antigen, or epitopes of that antigen. As mentioned above molecules traditionally thought of as targets e.g. epitopes could also be mimicked by the methods of this invention, in this way an epitope would be the "parent molecule".

### Active region.

In general terms an active region is that region of the parent molecule which is responsible for the activity of the molecule. Often the active region is only a small proportion of the total molecule. Active regions include the active sites of catalytic molecules and enzymes and binding sites of antibodies etc. Typically the active region is involved in interaction with a target molecule.

In the case where the parent molecule is a protein the active region may include one or more looped sections ("loops") and each loop may contain several tens of amino acid residues. These loops arise from the folding structure of the protein. Loops may also be found in nucleic acid parent molecules, including nucleoproteins.

### Active chemical groups.

Active chemical groups are those chemical groups of the parent molecule which are associated with the activity of that molecule. These active chemical groups typically constitute a relatively small proportion of the total number of chemical groups in the active region.

For example, when the parent molecule is a protein, these active chemical groups would be individual amino acids located within the active region which have a direct role in the activity of that protein. In the case where the protein is an enzyme the active chemical groups would be those amino acid residues found in the active region which are directly involved in the reaction associated with that enzyme, e.g. in interaction with the substrate, or the transfer of atoms within the enzyme-substrate complexes. Where the protein is an antibody, the active chemical groups would be the amino acids which are involved in antigen binding.

In the situation where the known molecule is a protein, this aspect of the active chemical groups (amino acids) may be located apart in the primary sequence of the parent protein. For example, one or more of the amino acids identified for the simulation may be located on different strands of the parent protein according to the known structure.

In particular where the parent molecule is an antibody it is desirable that the mimetic includes active chemical groups which derive from both the light and heavy chains of that antibody, particularly from the complementarity determining regions of both the light and the heavy chains of the antibody. It is preferred that the mimetic includes the most active of the CDRs, usually this is CDR 3 of the heavy chain. Active chemical groups may also be found in the framework region of the antibody. Where groups are identified as "active", they would be included in the mimetic.

### Framework chemical groups.

As mentioned above, the active chemical groups usually constitute only a small proportion of the active region. The active region also includes chemical groups most of which are not directly involved in the interaction with a target. These groups are referred to herein as non-active framework chemical groups. Framework chemical groups generally maintain the active chemical groups in the correct orientation to carry out the activity of the molecule.

### Identification of chemical groups associated with activity.

According to this aspect of the invention, active chemical groups are identified by a combination of i)experimental selection of potentially active chemical groups and ii) computationally determining the accessibility of potentially active chemical groups in their locations in the parent molecule. A three dimensional model or X-ray structure of the parent molecule is required to determine the accessibility of the chemical groups.

### i) Selection of potentially active chemical groups on the basis of experimental data

The selection of active groups on the basis of experimental data may include generating experimental data, or utilising known experimental data. In the first case, it may comprise the stages of selecting a region of the parent molecule and altering the chemical groups of that region and investigating the effects of these alterations on the activity of the molecule. Specifically, this may include producing a library made up of sections of the active region, each section comprising a string of chemical groups. Next, the individual chemical groups are altered either by replacement, deletion or modification. This may be achieved by producing a further library of sections of the active region in which individual chemical groups have been altered.

In the situation where the parent molecule is a protein, this approach may involve the production of a peptide library encompassing at least the active region of the protein. This library may be produced by conventional chemical synthesis or by recombinant means. The modifications of the amino acids could be made either by chemical modification to the amino acid side chains, or by substitution, replacement or deletion of the amino acids by chemical or genetic means.
Substitutions may include conserved substitutions where amino acids are replaced by amino acids of similar chemical nature and non-conservative substitutions where amino acids are replaced by amino acids of a dissimilar nature, as would be understood by one skilled in the art (discussed also later). Alternatively, any amino acid may be replaced by a small aliphatic amino acid, preferably glycine or alanine. For example, the alanine scanning method whereby amino acids are replaced by alanine may be used in the experimental determination of the potentially active chemical groups.

As noted above, the active groups to be included in a mimetic may be selected by either the selection methods described above or on the basis of existing data. In indeed, a particular mimetic may include residues selected by both of these approaches.

Examples of existing data upon which the active groups may be selected include the following: active groups may be selected for inclusion in a mimetic on the basis of structural data, e.g. X-ray crystallography or NMR; residues may be selected according to information from genetic databases, e.g. on the basis degree of conservation of chemical groups.

For example, in the case of certain enzymes it is known (from structural or genetic analysis) which amino acids are involved in binding the substrate. These residues could be included in a mimetic.

### ii) Computationally determining the accessibility of potentially active chemical groups in the parent molecule.

The degree of accessibility may be a numerical measure of the exposure of the chemical group within the parent molecule. In this case, the chemical group(s) associated with activity may be identified as those among the selected potentially active chemical groups for which the numerical measure is above a predetermined value.

For example, one suitable numerical measure of the degree of exposure is the surface area of the chemical group which is accessible to solvent.

Alternatively, the degree of exposure may be defined in terms of a "surface" of the active region of the parent molecule. For example, this surface may be defined as an envelope of the molecular motions of the active region (determined by a molecular dynamics simulation), or as a section drawn through the three-dimensional structure of the parent molecule to separate the most exposed portions of the parent molecule from the rest. The degree of exposure of a chemical group may then be measured in relation to this surface, e.g. as a distance to this surface, or according to whether the chemical group is (e.g. predominantly) on one side of the surface or the other.

### Molecular dynamics criteria.

Molecular dynamics criteria may be employed to determine which of a series of virtual mimetics (i.e. mimetics generated by computer rather than physically synthesised) have an arrangement of active groups most similar to the active chemical groups in the parent molecule.

In order to compare the folding structure of the mimetics with the parent structure, molecular dynamics simulations of regions of the parent molecule may be carried out. These molecular dynamics simulations can then be used for comparison with the molecular dynamics simulations of the mimetics. For example, simulations may be of selected loops which contain active chemical groups.

Mimetics can be screened on the basis of a predetermined criterion and varied to improve the degree to which the predetermined criterion is obeyed.

As a predetermined criterion, a first possibility is that the criterion indicates the flexibility of the mimetic (or a part of it) as determined by the molecular dynamics simulation. Preferably the mimetics should have flexibility characteristics similar to the flexibility characteristics of the corresponding region in the parent molecule.

Alternatively, or additionally, the criterion may be that the similarity between the time variation of the conformation of the potential mimetic with that of the parent molecule is above a predetermined level. For example, a principal component analysis may be performed of the motion over time of a section of the parent molecule (obtained by a molecular dynamic simulation) and of at least part of the mimetic. Similarity between these two time variations (as revealed by the principal component analysis) would suggest that the arrangement of the chemical groups in the mimetic is similar to the arrangement of the corresponding active chemical groups in the known molecule, and/or would suggest that the mimetic is relatively likely to exhibit the activity of the parent molecule.

In either case, it is possible that the whole of the potential mimetic is simulated. Alternatively, the dynamics simulation may be of a portion of the mimetic including a plurality of residues which have been identified as being part of the active region.

### Mimetic.

The term mimetic as used herein includes both physically existing (e.g. synthetic mimetics) and mimetics which exist only in a virtual sense in a computer simulation.

In this document, the term "mimetic" is used to include the case in which the mimetic shares the activity of the parent molecule but only to a lesser degree (for example, if the level of activity is represented by a numerical index, the value of the index for the mimetic may be only 50% that for the parent molecule) . Furthermore, the term "mimetic" would include even a case in which the mimetic actually has a greater activity than the parent molecule.

The mimetics of this invention can be of any chemical nature suitable to mimic the activity of the starting molecule. If the parent molecule is a protein then the mimetic would include active amino acid groups. Peptide mimetics are discussed in more detail below. It is preferred that the mimetic is short or small relative to the starting molecule. It is preferred that the mimetics of the invention have 5 to 50 amino acids, more preferably 8 to 40, even more preferably 10 to 30 amino acids.

For non-peptide mimetics, the size (e.g. defined as number of atoms) may be similar. For example, it may range from 5 to 60, or 8 to 50, or 10 to 40 amino acids. The range may be 500 to 6000 Daltons, preferably 800 to 5000 Daltons, more preferably 1000 to 4000 Daltons.

A mimetic may take the form of one or more loops including active chemical groups. Where the structure of the mimetic includes a loop, it is preferred that the mimetic is provided with a chemical linkage formed within the mimetic, for example a chemical bond arising from chemical groups within the mimetic, and does not require external templates. It is preferred that such linkages arise from framework chemical groups within the mimetic rather than from the active chemical groups. In the situation where the mimetic is a peptide such linkages include amide bonds and disulphide bridges or other bonds like ester, thio-ester, ether or thio-ether.

A mimetic may include two or more loops, which include active chemical groups. This may mean, if the parent molecule is a protein, that amino acid groups which are far apart in primary amino acid sequence are brought into close spatial relationship by appropriate linkages. Other examples of mimetic structures include a loop linked to linear peptide, two loops linked together, multiple loops linked together or a combination of loops and linear peptides linked.

### Linking structure.

Often a linking structure of some sort is required to maintain the mimetic in an appropriate arrangement. If a loop mimetic is appropriate, the linking structure can be a simple chemical bond arising from the chemical groups of the mimetic. The bond may arise from either the active or framework chemical groups of the mimetic. However, it is preferred that the bond arises from the framework chemical groups. Where the mimetic is a peptide a chemical bond may arise from the amino acid groups of the mimetic, e.g. from the side chains of those amino acids.

In the situation where a number of active chemical groups linked to maintain the geometry of those active chemical groups with a structure topologically dissimilar to the parent molecule, then the linking structure may be more than simple chemical bonds. In this situation the linking structure may include additional chemical groups (chemical linkage groups), from which chemical bonds arise. The linking structure is therefore a combination of the chemical linkage groups and the chemical bonds arising therefrom.

Generally, in either of the above situations the linking structure is varied to provide the appropriate arrangement of the active chemical groups in the mimetic. The variation could be by replacement, deletion or alteration of the bonds or chemical groups within the linking structure.

### Types of mimetics.

Peptide mimetics are discussed below. Peptoids are one example of non-peptidic mimetics. Unlike peptides, in peptoid molecules the side chain groups are bonded to nitrogen rather than to carbon. Peptoids may be synthesised by the polymerisation of N-substituted glycine residues. The N-substitutions can be any suitable side chain found in amino acids, so N-Ala would be glycine which is N substituted with CH₂ (See Simon, R J *et al.*, 1992, Peptoids: A Modular Approach to Drug Discovery, *Proc. Natl. Acad. Sci. USA,* 89:9367-71.

A peptoid backbone is shown below:

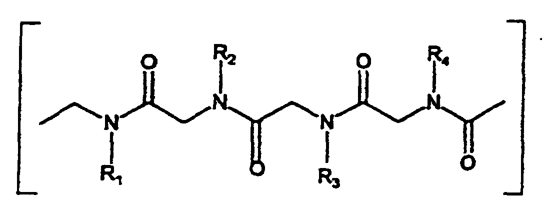

In addition to peptoids, other mimetics include pseudopeptides e.g., in which a modified amide bond is introduced, retro-inverso peptides e.g., in which the amide bond is inverted, and peptide bond mimetics. These mimetics are described in the following articles Emmons et al., Current Opinion in Biotechnology, 1997, 8: 435-441; Olson et al., Journal of Medicinal Chemistry, 1993, 8 (21):3039-3049; Fletcher and Campbell, Chem. Rev., 1998, 98: 763-795; Marraud et al., Biopolymers, 1993, 33: 1135-1148; Gante, Angew. Chem. Int. Ed. Engl., 1994, 33: 1699-1720.

Amino acids may also be connected by a carbohydrate backbone or any defined scaffold and template molecules.

Indeed, any suitable linking structure may be used provided the arrangement of active groups is suitable to mimic the function of the parent molecule.

### Peptide mimetics.

Except where specified to the contrary, the peptide sequences described herein are shown in the conventional 1-letter code and in the N- terminal to C-terminal orientation.

The term "comprising" means "including" and allows for the presence of further amino acid sequences, or other chemical moieties, at the N- and/or C-termini, provided that the peptide as a whole retains the activity of the parent molecule. Mimetics of the invention comprise the sequences, fragments thereof and variants of said sequences and fragments as set out above.

The invention also extends to fusion peptides comprising the mimetics described above, linked at the N- or C- terminus, or both, to further peptide sequence(s). These further sequence(s) may be selected to provide particular additional functions to the resulting fusion peptide. Such further sequences may be selected by those of skill in the art taking into account the intended purpose of the function.

The further peptide sequence may be a membrane translocation sequence capable of directing the fusion peptide through the membrane of a eukaryotic cell. Example of such peptides include the HSV-1 VP22 protein, the HIV Tat protein or portions thereof or a sequence that is derived from the *Drosophila melanogaster* antennapedia protein. The latter is a peptide containing 16 amino acid residues taken from the third helix of the antennapedia homeodomain protein which translocates across biological membranes. Other membrane translocation sequences known in the art may be used in an analogous manner.

A further class of sequences are tags which allow the detection of the peptide or its recovery by, for example, affinity chromatography. Many such tags are available and include the T7 tag, the HA tag and a myc tag.

A further class of sequences may provide effector functions, for may be a portion of the constant region of an antibody and may provide antibody effector functions.

In general the further sequence(s) will not comprise more than a total of 500 amino acids, optionally split between the N- and C-terminus in any proportion. More desirably the sequences will be much shorter, for example not more than 200, preferably not more than 100, for example not more than 50 or even not more than 20 amino acids in total.

### Production of peptide mimetic.

Peptides mimetics may be made synthetically or recombinantly, using techniques which are widely available in the art. Synthetic production generally involves step-wise addition of individual amino acid residues to a reaction vessel in which a peptide of a desired sequence is being made. Examples of recombinant techniques are described below.

To produce a peptide mimetic by recombinant means, polynucleotides encoding the peptide can be incorporated into a recombinant replicable vector. The vector may be used to replicate the nucleic acid in a compatible host cell. Suitable host cells are described below in connection with expression vectors. Preferably, a polynucleotide encoding the peptide designed using the invention in a vector is operably linked to a control sequence which is capable of providing for the expression of the coding sequence by the host cell, i.e. the vector is an expression vector.

The term "operably linked" refers to a juxtaposition wherein the components described are in a relationship permitting them to function in their intended manner. A control sequence "operably linked" to a coding sequence is ligated in such a way that expression of the coding sequence is achieved under condition compatible with the control sequences.

Such vectors may be transformed into a suitable host cell to provide for expression of a peptide designed by the invention. A process for preparing peptides designed by the invention comprises cultivating a host cell transformed or transfected with an expression vector as described above under conditions to provide for expression by the vector of a coding sequence encoding the peptides, and recovering the expressed peptides.

The vectors may be for example, plasmid, virus or phage vectors provided with an origin of replication, optionally a promoter for the expression of the said polynucleotide and optionally a regulator of the promoter. The vectors may contain one or more selectable marker genes. for example an ampicillin resistance gene in the case of a bacterial plasmid or a neomycin resistance gene for a mammalian vector. Vectors may be used *in vitro*, for example for the production of RNA or used to transfect or transform a host cell.

Host cells may be transformed or transfected with the vectors for the replication and expression of polynucleotides designed by the invention. The cells will be chosen to be compatible with the said vector and may for example be bacterial, yeast, insect or mammalian.

Promoters and other expression regulation signals may be selected to be compatible with the host cell for which the expression vector is designed. For example, yeast promoters include S. cerevisiae GAL4 and ADH promoters, S. pombe nmtl and adh promoter. Mammalian promoters include the metallothionein promoter which is can be included in response to heavy metals such as cadmium. Viral promoters include the SV40 large T antigen promoter, retroviral LTR promoters and adenovirus promoters. All these promoters are readily available in the art.

### Modifications of peptides.

As mentioned earlier the amino acid sequence of peptides designed by the invention may include non-naturally-occurring amino acids. When the peptides are produced by synthetic means, such amino acids may be introduced during production.

The peptide may also be modified following either synthetic or recombinant production, e.g to improve stability or activity of the peptide. For example, side-chain modifications for amino acids may be made to the side chains of peptides designed by the present invention using techniques known to those skilled in the art. Such modifications include for example, modifications of amino groups by reductive alkylation by reaction with an aldehyde followed by reduction with NaBH₄, amidination with methylacetimidate or acylation with acetic anhydride. The guanidino groups of arginine residues may be modified by the formation of heterocyclic condensation products with reagents such as 2,3-butanedione or glyoxal. Sulphydryl groups may be modified by methods such as carboxymethylation. The carboxy terminus and any other carboxy side chains may be blocked in the form of an ester group, e.g. a C₁₋₆alkyl ester, biotin fluorescent dyes, fluorescein and rhodamin.

### Formulations.

Peptides designed by the invention may be formulated in the form of a salt. Salts of peptides designed by the invention which may be conveniently used in therapy include physiologically acceptable base salts, eg derived from an appropriate base, such as alkali metal (e.g. sodium, potassium), alkaline earth metal (e.g. magnesium) salts, ammonium and NR₄ (wherein R is C₁₋₄ alkyl) salts. Salts also include physiologically acceptable acid addition salts, including the trifluoroacetate, hydrochioride and acetate salts.

Peptides designed by the invention may be in a substantially isolated form. It will be understood that the peptide may be mixed with carriers or diluents which will not interfere with the intended purpose of the peptide and still be regarded as substantially isolated. A peptide designed by the invention may also be in a substantially purified form, in which case it will generally comprise the peptide in a preparation in which more than 90%, e.g. 95%, 98% or 99% of the peptide in the preparation is a peptide designed by the invention.

A peptide designed by the invention may be labelled with a revealing label. The revealing label may be any suitable label which allows the peptide to be detected. Suitable labels include radioisotopes, e.g. ¹²⁵I, enzymes, antibodies, polynucleotides and linkers such as biotin. Labelled peptides may be used in diagnostic procedures such as immunoassays in order to determine the amount of a peptide mimetic in a sample.

A peptide or labelled peptide designed by the invention or fragment thereof may also be fixed to a solid phase, for example the surface of an immunoassay well or dipstick.

Such labelled and/or immobilized peptides may be packaged into kits in a suitable container along with suitable reagents, controls, instructions and the like.

### Pharmaceutical compositions.

Peptides designed by the invention may be formulated into pharmaceutical compositions. The compositions comprise the peptide together with a pharmaceutically acceptable carrier or diluent. Pharmaceutically acceptable carriers or diluents include those used in formulations suitable for oral, topical, or parenteral (e.g. intramuscular or intravenous) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

For example, formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents, and liposomes or other microparticulate systems which are designed to target the peptide to blood components or one or more organs.

Suitable liposomes include, for example, those comprising the positively charged lipid (N[1-(2,3-dioleyloxy)propyl]-N,N,N-triethylammonium (DOTMA), those comprising dioleoylphosphatidyl-ethanolamine (DOPE), and those comprising 3β[N-(n',N'-dimethyl-aminoethane)-carbamoyl]cholesterol (DC-Chol).

Compositions may comprise any desired amount of a peptide designed by the invention. In part this will depend upon the intended formulation and its intended use. By way of general guidance the composition may comprise from about 1% to about 99%, for example from 10% to 90% of a peptide designed by the invention.

The composition may comprise a mixture of more than one, for example two or three, peptides designed by the invention.

The invention will now be described in detail, with reference to a particular example which relates to the production of a peptide mimetic of an antibody. The methods used are applicable to the design of mimetics of antibodies in general, based on the methodology described herein.

In some antibodies, the VH chain alone is sufficient to provide binding to an antigen, and some species (e.g. camels) naturally generate single chain antibodies of this type. The present invention may be used to provide mimetics of VH single chain antibodies or the binding region of an antibody based on the interaction of a VH and VL chain, and reference to generating a mimetic of an antibody is intended to cover both possibilities.

In outline, to obtain a mimetic of an antibody, the starting point is to provide a three-dimensional structure of the target antibody. If no x-ray structure is available, a structure prediction method is required. Predicting the structure of the antibody variable region from sequence has been a subject of considerable activity since the work of Kabat and Wu (Proc. Natl. Acad. Sci. U S A 69:960-964, 1972; prediction of a kappa light chain), Padlan et al. (Cold. Spring. Harb. Symp. Quant. Biol. 41:627-637, 1977), Stanford and Wu (J. Theor. Biol. 88:421-39, 1981) and Feldmann et al. (J. Mol. Immunol. 18:683-698, 1981).

Following the essentially "homology" based predictions of these early approaches, methods were developed that introduced more rule-based procedures exemplified by the work of Rees and coworkers (Darsley and Rees, EMBO J. 4:383-392, 1985; de la Paz et al., EMBO J. 5:415-425, 1986; Martin et al., Proc. Natl. Acad. Sci. USA, 68:9268-9272, 1989 & Methods Enzymol. 203:121-153, 1991; Pedersen *et al*., 1992, Immunomethods 1:126-136; Rees *et al*., 1996, in "Protein Structure Prediction". Oxford University Press, edited by Sternberg MJE), and that of Chothia, Lesk and colleagues (Chothia *et al*., J.Mol.Biol. 196:901-917 1987; Nature 342:877-883, 1989; J.Mol.Biol. 227:799-817, 1992; Tomlinson *et al.,* EMBO J. 18:4628-4638, 1995; Al-Lazikani *et al*., J. Mol. Biol. 273:4 927-48, 1997) who, building on the observations of Kabat *et al.* (Ann. N. Y. Acad. Sci. 169:43-54, 1970; J. Biol. Chem. 252:6609-6616, 1977) and Padlan and Davies (Proc. Natl. Acad. Sci. U S A 72:819-823, 1975), developed the concept of canonical classes for certain of the variable region CDR's. Since then, attention has focussed on the more difficult problem of non-canonical CDR's, of which the antibody heavy chain CDR3 (hereafter referred to as H3) is the most non-conformist.

Antibody modeling has an advantage over protein modeling in general in that only the Fv needs to be modelled, the constant region being conserved. Further, the majority of the Fv itself, the framework, is highly conserved in structure between different antibodies and can be modelled using the most sequence-homologous known framework (Pedersen *et al*., 1992, *ibid*). For CDR modeling, 5 of the 6 CDRs (all except H3) frequently fall into one of between 1 and 10 canonical classes, a set for each CDR. Members of a canonical class all have approximately the same backbone conformation. This is determined by the loop length and the presence of a number of key residues, both in the CDR and the framework, which hold the CDR in a given conformation by hydrogen bonding, electrostatic and/or hydrophobic interactions. So, to model an unknown CDR, the sequence is examined, the appropriate canonical class assigned, and the most sequence-homologous known CDR used. For each loop except L2, a few examples fall outside existing canonical classes, and, along with the H3 loop, must be modelled in other ways (see http://antibody.bath.ac.uk). It is likely that further canonical classes will be revealed as more crystal structures are solved, although to date no strictly "canonical" classification has been possible for H3.

The difficulty of modeling the H3 loop is due to the extensive variability in both sequence and structure between different antibodies. There are essentially three approaches: knowledge-based methods, such as database searching, where the closest matching database loop either from antibodies, or from the entire Brookhaven Protein Data Bank (PDB; Bernstein *et al*., Eur. J. Biochem. 50:319-324, 1977) in sequence and length is used as the model, or ab initio methods, such as the CONGEN conformational search (Bruccoleri and Karplus, 1987(Biopolymers 26:137-168), or a combination of both (Martin *et al*., 1991, *ibid*). More recent analysis suggests that eventually, knowledge-based methods will provide the degree of accuracy required for most applications of modeling, such as antibody humanization (Reichmann *et al*., Nature 332: 323-327, 1988; Roguska *et al*., 1996, Protein. Eng. 9:895-904). However, for the time being, a combination of empirical and ab initic methods is likely to be necessary where accuracy approaching that of a medium resolution x-ray structure is required.

Having provided a three-dimensional structure of a starting antibody, two further items are provided, in any order. A section of the antibody which binds the target antigen is identified. For example, this is generally part of the surface of the antibody in which a number of residues having accessibility to solvent and/or the target are located.

In addition, the residues of the parent molecule associated with target binding activity are identified. Most conveniently, this can be achieved empirically, e.g. by alanine scanning, as discussed above. However, where models of the antibody bound to the target or structures of this are available - e.g. an x-ray crystal structure of the bound antibody, these may be used to determine these amino acids. It will be appreciated from the present description and the accompanying example that residues in the parent molecule associated with target binding activity, and indeed essential to the structure and function of the parent molecule include residues which are not directly accessible to the antigen, and are not essential for incorporation into a mimetic.

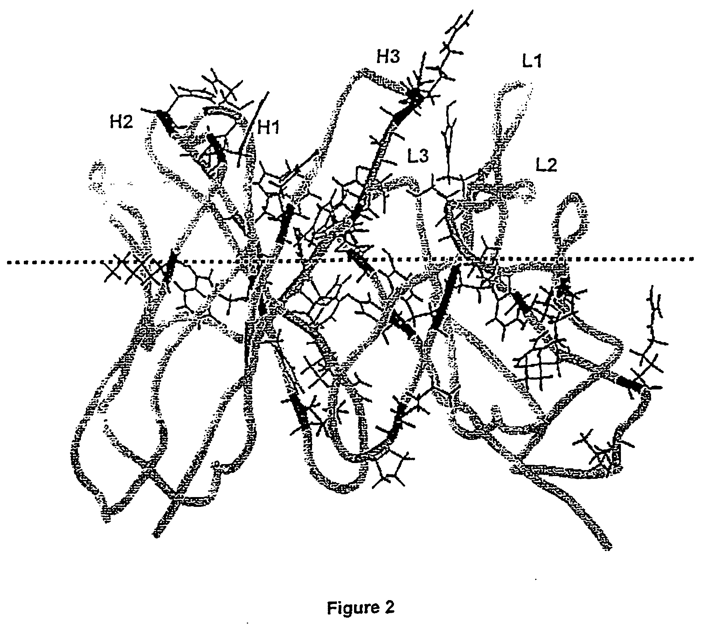

The amino acids associated with target binding activity may then be mapped onto the antibody model, and those amino acids which are located in the target binding region identified (for example, as shown in Figure 2).

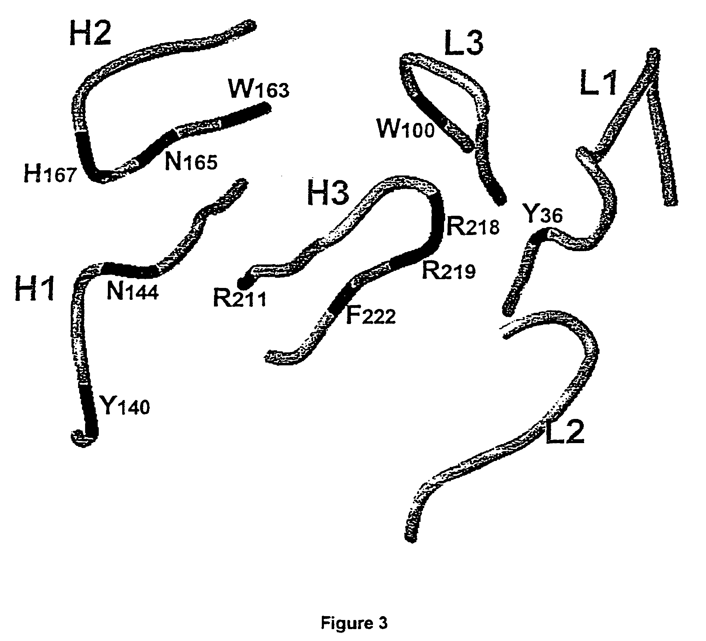

Having identified amino acids which are also in a target binding region, these can be extracted to provide a model (see for example Figure 3) which defines the three dimensional relationship between the individual extracted amino acids.

The skilled person will appreciate that it is not necessary to extract all the amino acids which are associated with target binding activity of the parent molecule and accessible to the target (e.g. by being at the binding surface of the molecule). For example, the binding surface may comprise two sub-regions which bind the target, in which case the amino acids of only one region may be extracted.

For experimental convenience, it may also be the case that a few target binding amino acids are discarded, to enable a mimetic of a more suitable size to be produced. For example, it may also be determined experimentally that some amino acids contribute to binding to a much higher degree than others. Thus it is possible to define an "energetic paratope" of the antibody binding region from which a discrete selection of amino acids for extraction are provided.

For example, the VH CDR3 (H3) loop of an antibody is often associated with strongest binding activity to a target, and it may be desirable to keep the target-binding residues of this loop in the mimetic. In the accompanying example, it will be seen that four such residues are present in the H3 loop, which is more than any of the other loops. In selecting the amino acids, this loop has been placed centrally in the group of amino acids to be selected, and the co-selected amino acids are set around the H3 amino acids. More generally, the selection may be based around a loop or loops which have the highest number of target binding amino acids, allowing one or more of the more distant target binding amino acids to be discarded. It is desirable that from about 4 to 15, such as from 6 to 12 amino acids are selected.

Desirably, the amino acids are selected from at least 2, preferably at least 3 and, in the case of a two chain (VH - VL antibody, more preferably at least 4 loops of the antibody.

Having defined the selected amino acids, a mimetic which incorporates these amino acids can be designed.

Desirably, the mimetic may be at least partially cyclic, i.e. the majority of the selected amino acids will be in a cyclic portion of the mimetic, for example no more than 1, 2 or 3 of the selected amino acids will be in a non-cyclic portion.

Linkers between the selected amino acids are chosen to maintain the geometric relationship between the selected amino acids. The linkers may be peptide linkers.

In one embodiment, the linkers may be refined by an iterative process of running a MD simulation of a first mimetic, determining the flexibility profile of the mimetic compared to the parent antibody, for example using the criteria illustrated in the accompanying example, replacing one or more linker molecules and determining if the MD simulation provides a mimetic which has improved flexibility properties (i.e. is more similar to the parent antibody).

In the first instance, the first mimetic may be designed by modelling linking groups between the selected residues which reflect to the extent practicable the structure or packing of the space between the extracted residues found in the parent molecule. Where the space between two selected residues can not be mimicked in this way, glycine residues may be inserted to maintain distance or flexibility. Proline residues may be provided to introduce turns or to control flexibility. This process will usually be performed manually or semi-manually on computer modelling software to provide a starting mimetic. The starting mimetic is then refined by the iterative process of the preceding paragraph.

This iterative process may be repeated until a linker with properties acceptable to the skilled person is obtained. Ultimately, what is acceptable will be determined experimentally, though the process will allow candidate mimetics to be designed which have a reasonable prospect of showing activity. For instance, for an MD of 100-500 ps on a standard modelling package such as AMBER, a RMSD of the mimetic from the original antibody structure of 10 Å or less, such as 5Å or less will be a favourable indication of activity which may be biologically relevant, and thus may form the basis for the actual synthesis and testing of the molecule.

Conveniently, the linkers are other amino acids, though other types of linkers, as discussed above, are also contemplated.

An advantage of the present approach is that the mimetic reproduces the binding section of the parent molecule in a compact structure which does not need to be carried by a base, as in the TASP approach discussed above. In the peptide format of mimetic in particular, the present invention provides for a mimetic in which the target-binding residues are located in a polypeptide molecule which is unbranched (apart from the cyclic linkage).

Another advantage is that the relative order of the selected amino acids in the mimetic is unconstrained by their natural order in the antibody molecule. For example, in the ST40 mimetic described herein, the amino acids selected from the H2 chain occur in reverse order. This provides for the mimetic to be smaller than would be the case if the amino acids were linked in linear order, even allowing for deletions between the selected residues.

As indicated above, the second mimetic may be'subject to further rounds of variation in the linking structure and MD analysis in order, to provide further mimetics which better obey predetermined criteria. The second or subsequent mimetic may then be synthesised and used as described herein.

As indicated above, the selecting may be based upon identifying the CDR loop with the greatest number of residues (or greatest equal number of residues) accessible to the antigen and identified as associated with target binding activity in the parent molecule, and selecting those together with residues from at least one other CDR. Preferably, the CDR loop with the next greatest number of residues (or an equal number, as the case may be) is the at least one other source of the residues.

Thus the invention provides a method for the design and synthesis of a pepricie which mimics the activity of an antibody, in particular the invention provides a method for the design and synthesis of a peptide which binds the CD4 receptor, e.g the CDR3 receptor of the CD4 protein. Those skilled in the art would be able to apply this approach to other antibodies to. design and synthesise peptides which would bind the targets of those antibodies. Examples include mimetics of antibodies against other receptors implicated in HIV infection, such as CCR5 and mimetics of antibodies against tumour necrosis factor.

As explained above these mimetics need not be designed based upon a natural occurring antibody, or even a synthetic antibody. In fact, the mimetics could be designed on the basis of a structure of a peptide selected from a peptide library (e.g. a phage display library), for example a peptide may be selected from a phage display library on the basis of its ability to bind a target, e.g. CD4. A mimetic could be produced based on this peptide and the resultant mimetic would bind CD4.

Accordingly, the invention provides a method for the design and synthesis of a peptide which binds CD4.

The mimetic peptides designed by the invention can be used in diagnostic techniques, e.g. detecting cells bearing the CD4 on their surface.

The CD4 binding peptide may be included in pharmaceutical compositions with a pharmaceutically relevant carrier.

The peptides may be used in the preparation of pharmaceuticals for the treatment of conditions in which CD4 is implicated. An example of such a condition is HIV.

Examples of the design and synthesis of peptide.mimetics of ST40 antibody will now be described in detail.

In this example the part of an antibody which recognises an antigen (the paratope) was investigated and mimetics of this paratope designed. The mimetics should have a low molecular weight compared to the antibody and should be easy to synthesise.

Biological assays using overlapping synthetic peptides followed by alanine scanning define the amino acids which can play a role in the binding.

ST40, an antibody that recognises CD4 and is a neutralising antibody in a cell-based HIV infection model, was. selected to start the project and to validate the method. This antibody was chosen because it is well studied. The three-dimensional structure of ST40 has been modelled with the AbM software (research version). The amino acids predicted by the Alascan which are located in the CDRs (where the binding occurs) and with a good accessibility are selected to be included in the mimetic. During the building of the mimetic, the location in space, the orientation and the conformation of all important amino acids are conserved as much as possible, especially their side chains. A molecular dynamics (MD) simulation in water of the CDRs of the mimetic and the parent antibody was carried out to define their flexibility.

Two methods of predicting and providing mimetics were used.
1) The three-dimensional structure of ST40 was modeled using the research version of the AbM (Oxford Molecular, UK) software. The amino acids predicted to be active by alanine scanning (Monnet *et al.,* 1999, J.Biol.Chem. 274:3789-3796), and which were located in the CDRs with good accessibility, were selected to be included in the mimetic. During the building of the mimetic, the location in space, the orientation and the conformation of all 'active' amino acids were conserved as much as possible, particularly the side chains. An oligopeptide mimetic containing only those CDR residues identified as critical for binding was designed. These critical amino acids were linked together by other amino acids and then cyclized at a carefully selected position in the sequence. Since this mimetic design was a continuous sequence, it does not have an exact structural similarity with the discontinuous CDR loops of the paratope. The flexibility and conformation of various versions of this mimetic design were evaluated by analysis of molecular dynamics simulations in water. The peptide mimetic of ST40 selected as active was synthesized and tested for its binding affinity and its biological activity (HIV retroviral inhibition). A cyclic peptide with the same number of amino acids as the mimetic and containing the important amino acid residues, but located at different positions in the peptide, was used as a negative control.
2) Three types of mimetics were simulated containing a number of CDR loops linked together at various points to form pseudo-cyclic structures. The number of CDRs varied from 1 to 3, so as to reduce the number of amino acid residues in the mimetics. The mimetics contain only amino acids (but it could be other types of molecules in the future) with specific cross-links made via the side chains of Lys, Glu or Cys. Their flexibility and global conformations are defined by molecular dynamics simulations in water, with or without ions followed by analysis with Principal Components Analysis (PCA). The PCA is based on the MD trajectories of the relevant molecules and is compared to the PCA calculated from the MD behaviour of the intact Fv region of the antibody.

### Material and Analysis Methods.

### (A) Antibody Modelling.

The cloning or MAb ST40 and its Alanine scanning is described in Monnet *et al*., 1999, *ibid*.

Molecular modeling of the light and the heavy chains of the antibody, especially the 6 CDRs (L1, L2, L3 (for the light chain), H1, H2 and H3 (for the heavy chain)) involved in antigen (CD4), recognition was carried out with the research version of the antibody modeling software AbM (Rees *et al*., University of Bath, UK) running on a 02 R5000 Silicon Graphics workstation. The AbM software has been developed specifically to model antibodies by a combination of homology rules and conformational search, starting with the primary amino acid sequences (Martin *et al*., 1989 (*ibid*); Pedersen *et al*., 1992, *ibid*). The latest version of AbM, used in this study, was developed at the University of Bath and contains a database of approximately 80 X-ray structures of antibodies. The L2, L3 and H1 loops which have a standard size were constructed using the canonical Classl frameworks and canonical Class 2 for H2, as defined in A*b*M. A new canonical class had to be defined for the unusually long L1 CDR loop of ST40 containing 15 amino acids. Four X-ray structures of antibodies (libg, 1mf2, lacy, and 1ggc) with L1 of 15 residues were identified and superimposed. Each of these L1's had a similar conformation. On the basis of this, the following canonical class for L1 was defined and the X-ray structures missing in the database were added: [TI]-x(20)-C(1)-x(1)-A-x(3)-V-x(7)-S-x(1)-[MLI]-x(1)-W(1)-x(35)-F (Searle *et al*., 1995 (Antibody structure and function. *Antibody engineering, edited by Borrebaeck, C.A.K., Oxford university press* 3-51)). The H3 loop of 13 amino acids, which is too long to fit into any H3 classification (Shirai *et al.,* 1996 (FEBS Lett 399:1-8); Shirai *et al.,* 1998 (J. Mol. Biol. 278:481-496)) was built using the conformational search program CONGEN (Bruccoleri and Karplus, 1987, *ibid*) implemented in A*b*M, combined with a 3D structural database search. The definition of the building blocks to generate H3 with CONGEN was modified several times to obtain 4 different models since the 'kinked' feature was not very well defined. The X-ray structure of the antibody libg, containing an L1 and a H3 loop of 15 and 13 residues, was then used to model H3 of ST40 with the same conformation. This model of H3 shows a kink and an extended form. Modeling was achieved through simple mutations and the structure was fully minimized using the Tripos force field. Hydrogens were added to all models of ST40 using the Sybyl software (Tripos, Inc.) and the models were minimized during 100 iterations with the conjugate gradient method to eliminate all small steric conflicts.

### (B) Modelling of the Mimetics.

All the molecules were visualized and modified using the Sybyl software running on an O2 R5000 Silicon Graphics workstation. For the modeling of the mimetics, CDRs were selected from the antibody, maintained in their original conformations and then linked together with chemical linkers via appropriate side chains and minimized. Molecular Dynamics (MD) simulations of the mimetics were carried out in water with the AMBER force field using the AMBER software (Oxford Molecular) running on a 0200 R10000 Silicon Graphics workstation using 4 processors. The input files for AMBER were prepared with the XLEAP module. The cross-linking bridge, -CO-NH-, between side chains was defined as an amide bond. The water box was calculated with the module SolvateBox with a box size of 8 Å. The water boxes contained between 2500 and 3500 water molecules for all the simulations and were generated with the WATBOX216 module. This corresponds to a Monte Carlo distribution of water with periodic conditions and constant pressure. The temperature was fixed at 300K, the cut off was set at 10 Å, the dielectric constant was set at 1 and the sampling frequency was ips. Before running the dynamics, the solvent molecules were minimized and then the solute. The MD simulations were run for 100 or 300ps. The first 20ps of the dynamics were the heating period, during which the system was taken from 10K to 300K, and it was then increased by 15K every 1ps.

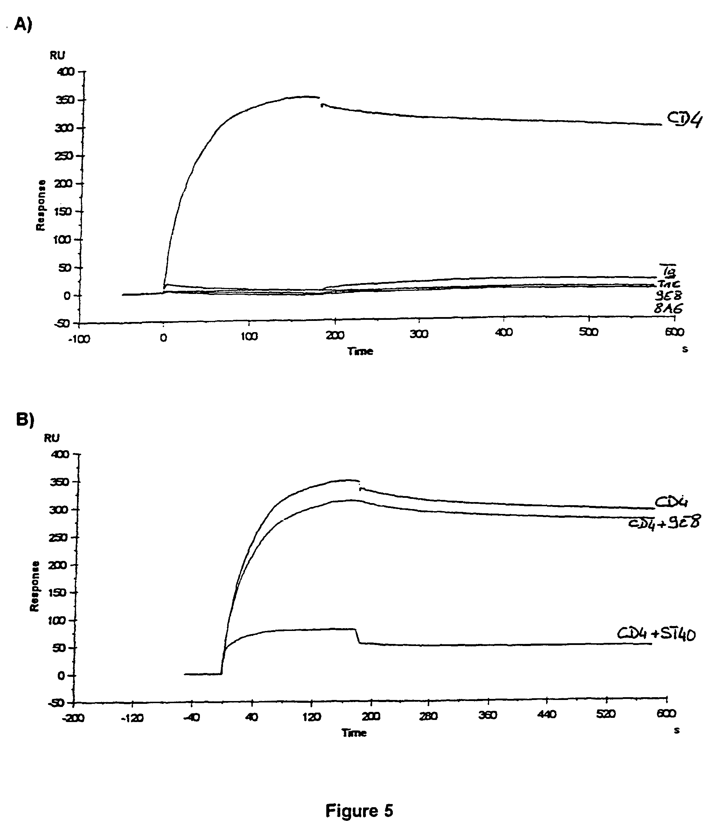

For the MD simulation of the antibody ST40, only the CDRs plus some framework amino acids were simulated, while the rest of the Fv was deleted (see Figure 5). Again, only the H3, L1 and L3 loops were allowed to move during the simulation since these were the selected CDRs for the mimetic. Two MD simulations were run, one without ions and one with 3 Na+ ions added around the H1, L1 and H3 loops. All other charged residues except those of H3L1L3 were neutralised during the simulation with ions. The simulation time was 300ps.

### C) Analysis of the MD Simulation.

The first 50ps of the dynamics simulations were not taken into account during the analysis since it was taken as the equilibration time.

The flexibility of the mimetics was compared to the parent antibody by different methods:

First the mimetics at different times during the dynamics simulations were superimposed onto the starting conformer which was very close to the conformation in the antibody. This displays quite well the deformation of the mimetics during the MD simulation and was carried out with the SYBYL software.

Then the RMSD's were calculated, using the starting conformer as a reference, for each ps of the MD simulation with the CARNAL module from the AMBER package (Oxford molecular). These were compared to the same data calculated for the MD simulation of the antibody.

Finally to describe more efficiently the MD simulation of the mimetic in comparison with the antibody, Principal Component Analysis (PCA) of the autocorrelogram (distance matrix) of the backbone of the mimetic was calculated at each ps. The autocorrelogram of the corresponding loops in the antibody was used as a reference. The program Anadyn, developed by Synt:em, allows the calculation of the autocorrelogram for specific amino acids and also for fragments of the backbone of the molecule. For this study, only the backbone of the specific amino acids were taken into account to eliminate the problems of mutated amino acids. The PCA analysis was carried out with the program TSAR (Oxford molecular).

### (D) Modelling of ST40

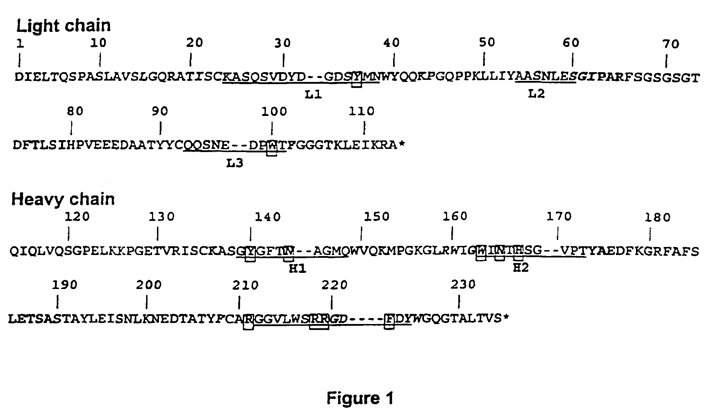

The alignment of the amino acids sequence of ST40 and the definition of the CDRs in AbM are shown in Figure 1. ST40 contains two unusually long CDR loops, L1 (15 residues) and H3 (13 residues).

The CDRs L1, L2, L3, H1 and H2 were all defined by canonical classes using the homology modelling with AbM. In contrast, H3, which is more flexible, was modelled using Congen, a conformational search method. The definition of the building block to generate H3 with Congen was modified several times to obtain 4 different models. Unfortunately, the kink was not very well defined in these 4 models. The X-ray structure of the antibody libg, containing an L1 and an H3 loop of 15 and 13 residues, was then used to model H3 cf ST40 with the same conformation. This model of H3 shows a kink and an extended form. Modelling was achieved through simple mutations and the structure was fully minimised using the Tripos force field. Hydrogens were added to all models of ST40 using Sybyl and the models were minimised during 100 iterations with the conjugate gradient method and the Tripos force field, to eliminate all steric conflicts.

The new canonical class identified by us for L1 permitted the construction of this loop in a specific conformation, while for the H3 loop several possible conformations were identified. Only four X- ray structures of antibodies containing H3 loops of 13 residues have been described, each having a different conformation. A comparison of the 3D structures of H3 loops containing 11, 12, 13, 14, 15 and 16 residues was carried out and showed that the highest variability occurs at the apex of the loop. However, when the sequence contains an Arg at position N-1 of H3 and Asp-x-Trp at the C-terminus, as in ST40, a kinked form (Shirai *et al*., 1996, *ibid*; Shirai *et al*., 1998, *ibid*) is observed at the C-terminal end of the loop. This has been observed in the antibody libg, which contains an L1 and an H3 loop of 15 and 13 residues respectively. The selected 3D structure of H3 in ST40, containing an extended terminal part of the loop and a kink near the C-terminus, is shown in Figure 2. This structure was selected as the reference model for the design of ST40 mimetics. However, the inherent flexibility of these longer H3 loops suggested that, during the mimetic construction, care should be taken not to constrain this region of H3 too heavily.

### (E) Dynamics Simulation of ST40 in Water.

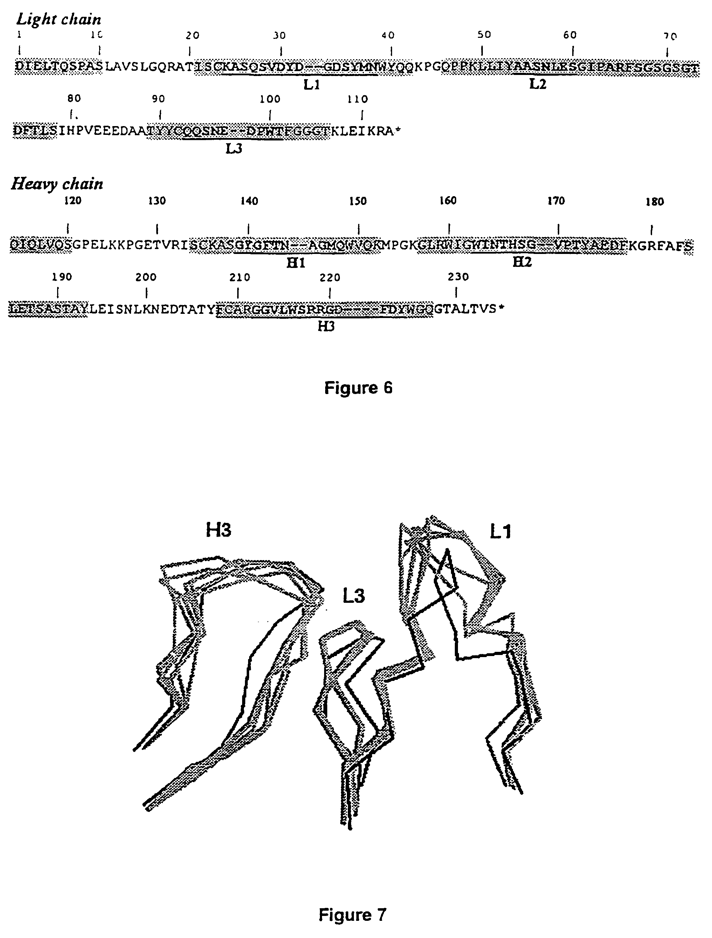

The fragments of ST40 selected for the MD simulation are displayed in grey on Figure 6.

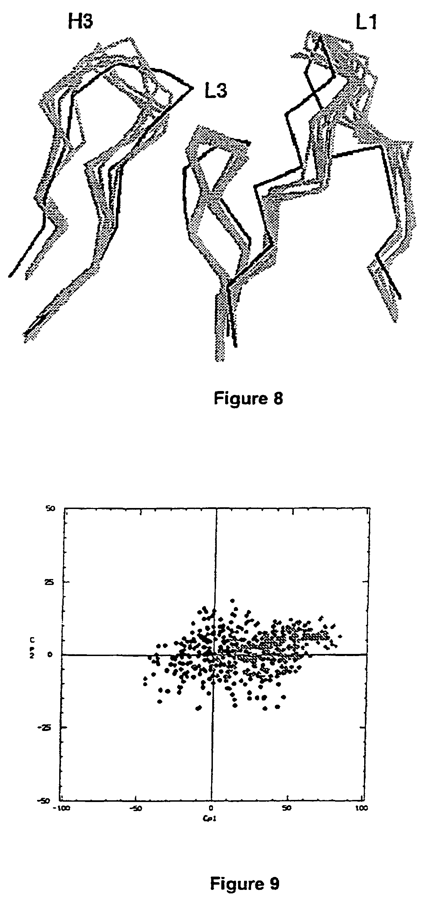

The superposition of the loops free to move is displayed in Figure 7 for the simulation without ions, and in Figure 8 for the simulation with ions.

The top of L1 had undergone large movements, as also observed in the mimetic, while L3 moved only at the beginning and then remained stable throughout the simulation. Interactions between H3 and L3 were observed.

The loop L1 moved differently to that observed during the MD simulation without ions and seemed more stable. H3 had less interaction with L3. The comparison between the two dynamics simulations using PCA is represented in Figure 8.

The MD simulation without ions is more compact than the MD with ions. This is probably due to the interaction between H3 and L1, which is less pronounced in the MD simulation with ions. The presence of ions induced dispersion. It should be noted that both H3 and L1 exhibit some flexibility, which should be taken into account during the design of the mimetics.

### (F) CD4.

The human recombinant CD4 used for the kinetic analysis and the biological assays was obtained from RepliGen (USA).

### (G) BIAcore Analysis.

The kinetic parameters, association rate constant (kₐ) and dissociation rate constant (k_{d}), were determined by surface plasmon resonance (SPR) analysis using BIACORE 2000 (Biacore AB, Uppsala, Sweden). kₐ and k_{d} were determined using BIAevaluation 3.0 software (BIAcore AB). The apparent equilibrium constant K_{D} is the ratio k_{d}/kₐ. All experiments were carried out at 25°C.

The free SH group of the lysine side chain extended by a 3-mercaptopropionic acid linker of the mimetic was used to chemically immobilize molecules on a B1 sensor chip (BIAcore AB) following a standard PDEA/EDC/NHS procedure from BIAcore.

The SPR signals for immobilized peptide mimetics were found to be about 280-500 Resonance Units (RU) after completion of the chip regeneration cycle, which corresponds to 280-500 pg/mm². The binding kinetic of CD4 to immobilized mimetic was determined by injecting CD4 (1-20 µg/ml, 16-330 nM) in HBS buffer (running buffer) at a flow rate of 30 µl/min.

For the selectivity study of the mimetic, the binding kinetics of immobilized mimetic were determined by injecting irrelevant proteins: 8A6 (anti-lyphocyte MAb), 9E8 (anti-troponine i MAb), Troponin C and Thyroglobulin (Tg), each at 50 µg/ml, in HBS buffer at a flow rate of 30 µl/min.

For the competition study, ST40 mAb (50 µg/ml - 330 nM) and CD4 (20 µg/ml - 330 nM) were pre-mixed, then co-injected on the sensor chip. The same experiment was carried out for the irrelevant protein 9E8 (50 µg/ml).

### (H) Anti-retroviral activity assays.

### (i): Isolation and culture of mononuclear cells from peripheral blood.

Mononucleated cells were isolated from peripheral blood of a healthy donor (and seronegative for HIV and hepatitis viruses) with a gradient of Ficoll-Hypaque. The PBMCs were activated by 1 mg/ml of phytohemaglutinine-P (PHA-P, Difco Laboratories, Detroit, USA) during three days, then cultured at 200,000 cells per well in a 96-well plate, in a culture medium A, complemented by 20 IU/ml of recombinant interleukin-2 (rHuIL-2, Roche Products, Mannheim, Germany). Medium A consists of: RPMI cell culture (RPMI 1640, Roche Products), 10% Fetal Calf Serum (FCS, Roche Products complement inactivated by heating at 56°C for 45mn, 2mM L-glutamine (Roche Products) and a solution of three antibiotics (penicillin, streptomycin and neomycin (PSN, LifeTechnologies, Grand Island, JSA). During culture, cells were maintained at +37°C, in a saturated humid atmosphere, under 5% CO₂.

### (ii): HIV Virus.

PBMCs were infected over 1 hour at 37°C with a 10 000 tissue culture infectious dose 50% (TCID50) of the lymphocytary tropism reference strain HIV-1-LAI. At the end of the incubation, cells were washed twice with medium A. The HIV-1 strain was exclusively amplified, in the Neurovirology Service, with Umbilical Blood Mononucleated Cells (UBMC) activated with PHA-P, and the TCID50 was calculated using the Kärber formula.

### (iii): Mimetic and control molecules.

The mimetic and the scrambled peptide were dissolved in ultrapure sterile water. Five concentrations were tested (50 µM, 10 µM, 5 µM, 1 µM and 0.5 µM). Antibodies ST40 and 9E8 (provided by the CNRS UMR 9921, Montpellier) were tested at the following concentrations: 100 µg/ml, 10 µg/ml, 1 µg/ml, 100 ng/ml and 10 ng/ml.

The activities of these compounds were compared to the Q4120 reference antibody and to AZT. These two latter compounds were provided by SpiBio (Paris, France). The Q4120 antibody was tested at the following concentrations: 5 µg/mL, 500 ng/mL, 250 ng/ml, 50 ng/ml and 25 ng/ml. The anti-HIV activity of AZT was measured at the following concentrations: 10 µM, 1 µM, 100 nM, 10 nM and 1 nM. All dilutions were performed in medium A.

### (iv): Antiviral activity assay on PBMCs infected by HIV isolates.

All experiments were performed in triplicate. PBMCs were pretreated for 1 hour at +4°C with the relevant molecule, then infected over 4 hours at +37°C with 10 000 TCID50 of the HIV-1-LAI strain. Cells were then washed twice with 100 mL of A medium. Cells were cultured in medium A supplemented with 20 IU/mL of rHuIL-2, and half of the culture medium was renewed on the 7th day of the culture. On days 7 and 10 of the culture, supernatant was removed and frozen at -20°C. The viral replication was measured by dosing the inverse transcriptase activation in the supernatant of the 7th day culture, using a dosing kit Retrosys(r), supplied by Innovagen (Lund, Sweden).

In parallel with this antiviral activity assay, the cellular viability was evaluated by microscopic observation. Any decrease of the cellular concentration, the presence of cellular fragments or a modification of the cellular morphology were logged. The cellular viability decrease was quantified with the exclusion dye, Trypan Blue.

### Example 1: Continuous Cyclic Mimetic.

To obtain a continuous peptide mimetic easy to synthesize, the selected amine acids from the CDRs (shown in Figure 3) were extracted. The important amino acid residues of ST40, defined by peptide scanning followed by alanine scanning by Monnet *et al.,* (1999, *ibid*), are shown in Figures 1 and 2. It was observed that some important amino acids are not located within, or in some cases even near, the CDR regions where the paratope is located (Figure 2). To select the amino acids that should be included in the mimetic, the bioactive residues were displayed on the 3D structure of ST40 (Figure 2) and their accessibility to water was calculated (Table 1). Some residues were found to be located at some considerable distance from the paratope binding surface (top of Figure 2), such as Lys152 and Lys43, whereas others were buried in the protein with poor accessibility to antigen. For example, many of the aromatic residues such as Tyr40, Phe102 or Trp149, often playing a structural role in all antibody variable regions, are amongst the buried residues identified as 'active' by the mapping procedure. In contrast, charged residues such as Arg218 and Arg219, more likely to be directly involved in the binding, were conserved. In addition to these hydrophilic residues, amino acids that were predicted to be essential for maintaining the hydrophobic stabilization of the mimetic and which were likely to exhibit increased accessibility in the mimetic because of the flexibility of the H3 loop (eg Phe222, Trp163 and Tyr36) were conserved, even though their measured accessibilities on the static model may have been low.

Finally, a limit was fixed for those amino acid residues that should be included in the mimetic based on their importance as defined by the alanine scanning protocol, and their likely accessibility to the antigen. The amino acids selected are boxed in Figure 1 and displayed on the loops in Figure 3. All CDRs except L2 are represented in the mimetic by at least one residue.

To obtain a continuous peptide mimetic amenable to synthesis, the selected amino acids from the CDRs (shown in Figure 3) were extracted. The relative locations and orientations in space of the selected amino acids residues, especially the side chains, were conserved and the amine acids were linked together using glycine residues to obtain a cyclic peptide. The cyclization is performed by a covalent bond between the NH2 of an internal lysine side chain and the C-terminal carboxyl of the peptide. This position was selected to ensure some flexibility within the N terminal segment of the mimetic which contain the two arginines derived from the flexible H3 loop of ST40. To stabilize the mimetic the glycine residues were then systematically replaced by hydrophobic residues, or proline residues for the turns, to obtain the most stable mimetic in which the important amino acids exhibited the same (or closely similar) orientations as observed in the intact parent antibody, ST40. After minimization and short MD simulations (100 ps) the positions and orientations of the side chains of the important residues were checked. The short MD simulation allows any 'strong' deformation of the mimetic to be observed. The most stable mimetic selected from the MD simulations for synthesis had the lowest RMSD (5 Å) after 100ps. This mimetic, named MK3Tyr, has the following amino acid sequence:

NHCO
AcNH2AR218R219PKF222YR211APY140VIN144H167PN165VW163GPW100VAY36GP

In this mimetic most of the side chains of the important amino acids maintain a correct conformations during the 100 ps of MD simulation. When the simulation is extended to 300 ps the RMSD increases to 5.49 (average of the last 100 ps) due to the inherent flexibility of the peptide.

To prove the importance of the location of the amino acids residues involved in the binding, a similar cyclic peptide was synthesized with the important residues at different positions and other amino acids for linkage than in the mimetic. This "scrambled" peptide has the following amino acid sequence:

NHCO
AcNH-IW100VKAPH167VPY36N165R219CF222[?]140GPR[?]APN144AR218PVW163G

The syntheses of the mimetics and the scrambled peptide were carried out in a stepwise fashion on an Automated Multiple Peptide Synthesis (AMS 422, ABIMED). HPLC analyses were carried out on a Beckman LC126 system, using Waters SymetryShield column RP18, 5 mm, 100 Å (150 x 4.6 mm) with buffers A : 0.1% TFA in water, and B : 0.08% TFA in acetonitrile; gradient from 95% A to 100% B in 12.5 mn with a flow of 1.5 ml/mn. HPLC purifications were carried out on a Waters Prep LC 4000 system, using Waters PrepPak Cartridge Cl8, 6 mm, 60 A (40 x 100 mm); gradient from A to 60% B in 60 mn with a flow of 20 ml/mn. Mass analyses were performed using a Maldi-tof spectrometer (Voyager DE Elite, PE Applied Biosystems) with dihydroxybenzoic acid as matrix.

The peptide synthesis was performed by a classic Fmoc solid support protocol. The synthesis was carried out using a polyethylene glycol graft polystyrene support (Fmoc-PAL-PEG-PS resin, substitution: 0.41 mmol/g). The Fmoc amino acids were activated in situ by the activating reagents DIPCDI (diisopropylcarbodiimide) and HOBt (1-hydroxybenzotriazole) in DMF (N,N-dimethylformamide)] with a standard fourfold excess. An acetylation step was carried out after each amino acid incorporation to cap possible remaining amine groups and to ensure the absence of deletion peptides. The Fmoc protecting groups were removed by a solution of piperidine in DMF (20%) prior to each coupling.

After cleavage of the support and removal of side chain protecting groups by reagent B, a standard TFA-based cocktail (88% TFA, 5% Phenol, 5% H2O, 4% TIPS [v/v]) and a post-cleavage work-up by ether precipitation, the purity of crude linear peptide was checked by analytical HPLC and mass spectroscopy. If purity was over 85%, crude linear peptide was cyclized at room temperature using 1 equivalent of PyBop and 5 equivalents of NaHCO3 in DMF. The reaction mixture was purified by preparative HPLC and the pure product lyophilized to obtain a white powder with a global yield from 5 to 20%.

If purity of the linear peptide was less than 85%, an intermediate purification by Preparative HPLC followed by lyophilization was achieved before cyclization.

In the case of the mutated mimetic with a lysine extended by a 3-mercaptopropionic acid linker instead of an isoleucine, the initial NH2 function of the side chain of this residue was specifically protected by the Alloc (Allyloxycarbonyl) protecting group that is specifically removed after the cyclization step according to the following conditions: Pd[PPh3]4 (3 equivalents), CHCl3 / AcOH/ NMM (37/ 2/ 1), r.t., 3 H. The 3-(tritylthio)propionic acid was then coupled to the free NH2 function using standard PyBOP activation in DMF. Cleavage of the support and removal of side chain protecting groups was achieved as described above.

The pure products were analyzed by analytical HPLC and Maldi-Tof mass spectrometry.

### Kinetic parameters.

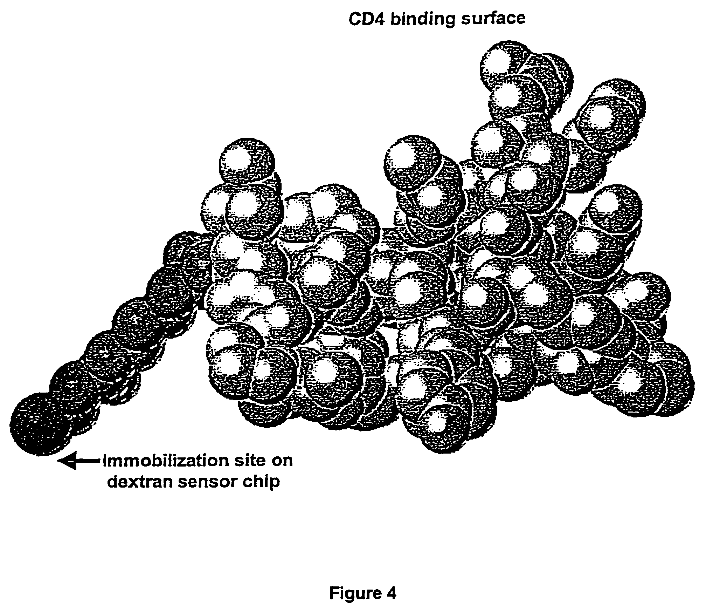

The mimetic MK3Tyr was bound covalently to the sensor chip and the CD4 was maintained in solution. The binding of the mimetic to the sensor chip required a free amine or sulfide group on the mimetic. One solution was to mutate an amino acid of the mimetic not involved in the binding to a Lys extended by a 3-mercaptopropionic acid group (3MP = COCH₂CH₂SH) to obtain a long linker. This mutation was chosen carefully because the surface of the mimetic interacting with the CD4 had to be accessible when the mimetic was fixed to the sensor chip. Based on the average structure of the last 100ps of the 300ps dynamics simulation, the Ile between Tyr140 and Asn144 has been identified as the best location for mutation. At this position the Lys side chain extended by 3MP was observed to be pointing away from the surface predicted to be important for the CD4 interaction. This design is represented in Figure 4 and the mimetic was named Mk3Tyr-long1SH.

The kinetic parameters, ka and kd, of the immobilized mimetic Mk3Tyr-long1SH and soluble CD4 were measured and gave values of ka 8.26 x 10⁴ s-1M-1 and kd 2.64 x 10⁻⁴ s⁻¹. The calculated K_{D} is 3.2 nM.

The interaction of several non-relevant proteins (8A6, an anti-lyphocyte MAb, 9E8, an anti-troponin I MAb, Troponin C and Tyroglobulin) with the immobilized mimetic was also tested and the results are shown in Figure 5A. No binding was observed for any of these proteins.

For the competition study, ST40 MAb (50 µg/ml) and CD4 (20 µg/ml) were pre-mixed, then co-injected on the sensor chip (Figure 5B). The same experiment was carried out with the irrelevant MAb 9E8. The binding of CD4 to the mimetic was inhibited by ST40, while the irrelevant MAb 9E8 had no effect on the binding. This clearly demonstrates that the mimetic bound to the same, or very closely located, site as ST40 on CD4.

### Anti-retroviral activity assays.

The anti-retroviral activity of the mimetic was tested in a cellular assay, as described in the methods section. The irrelevant antibody 9E8 was used as a negative control and ST40 as positives. The anti-HIV reverse transcriptase inhibitor, AZT, and the antibody Q4120 were used as the positive reference. The data are presented in Table 2.

The negative control antibody, 9E8, shows a dose independent activity of between -5% and +13% inhibition, considered to be within the range of background assay noise. The positive control antibody Q4120 and the mimetic parent antibody ST40 show similar dose dependent activities, giving 96% maximum inhibition. The mimetic shows a dose dependent inhibition, with a maximum of 70% viral inhibition at 50 µM. No cell death was seen during any of the assays. In contrast, the scrambled peptide shows a dose independent, non-linear behavior and, as with 9E8, is considered to be within the background noise.

### Example 2: Design of a Mimetic of ST40 Containing CDR Loops

The first step in the design was the selection of the amino acids of the Fv which should be included. The 5 selected fragments, containing a total of 36 residues, are displayed in boxes in Figure 6. The most difficult problem is to link these fragments of amino acids together and to retain the conformations of the CDRs relative to one another. If all 5 loops are included in the mimetic, the number of residues after linkage would be more than 60, which is not acceptable in terms of synthesis. For this reason, it was decided that mimetics of H3 alone would be modelled, synthesised and tested, then in a second step, mimetics of H3-L1 and finally mimetics containing all three loops H3-L1-L3. The loops were connected using the side chains of appropriately placed residues. Numerous mimetics were modelled but only few examples will be presented.

### A) H3 Mimetic.

All the H3 mimetics were linked at each end of the loop by an amide bond or by a disulphide bridge involving the side chains of Cys, Glu, Lys, Arg or Orn.

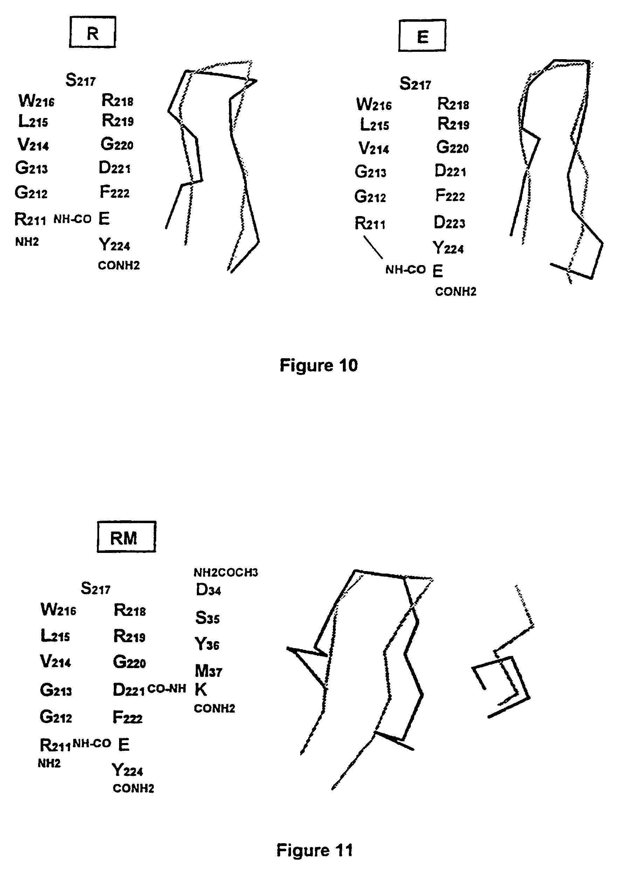

A MD simulation was carried out for six mimetics of H3. The results show that the mimetic with a disulphide bridge does not keep its starting conformation. The most stable mimetics for H3 are R and E, represented in Figure 10. These 2 mimetics were selected for the addition of L1. The mimetic E is preferred because the linkage does not involve an important amino acid. The mimetic E was synthesised.

### B) H3L1 Mimetic

The second fragment representing L1 (see Figure 1) was linked to H3 via the side chains of Asp221 and a Lys added on L1 by mutation of Asn38. The H3 mimetics R and E were both used for the modelling of H3L1.

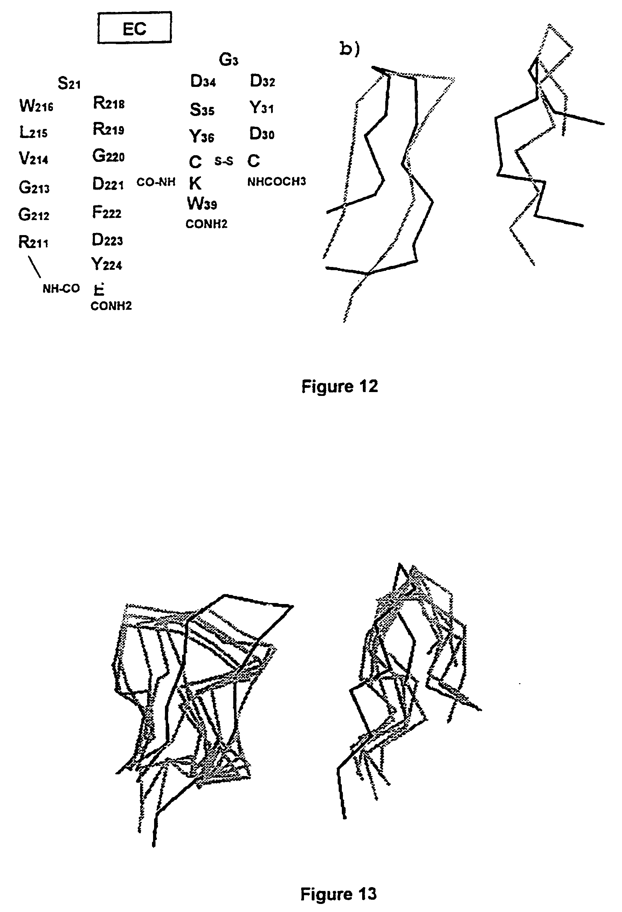

The first mimetic RM has a very short fragment for L1 (see Figure 11), which was very flexible and interacts with H3, inducing a distortion of this loop during the MD simulation. Then, a fragment of L1 was used (Va129 to Met37) and a disulphide bridge was included by mutation of Va129 and Met37 to Cys, to stabilise the conformation of L1. Trp39 was added to L1 to increase the number of aromatic residues at the bottom of the mimetic as in the parent ST40. The mimetic EC was more stable after 100ps dynamics simulation than RM even if L1 adopted a cyclic conformation at the top of the loop, in contrast with ST40 (Figure 12). This was not considered to be an issue since this part of the molecule is flexible during the MD simulation of ST40 itself, and in any case the fragment L1 should be more stable in the presence of L3. Hence no further version of the H3L1 mimetic was modelled.

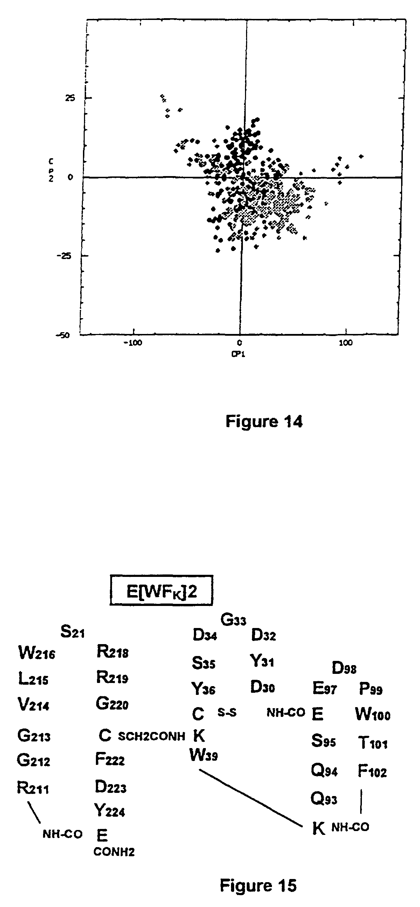

Finally, the linkage between E and C was modified to facilitate the synthesis. Asp221 of the E loop (H3) was mutated to Cys to form a linkage S-CH2-CO-NH with the Lys of C loop (L1), by the addition of I-CH2-CO-(N1-CO-CH2-CH2-C10). A MD simulation of 300ps of this mimetic in water with one Na+ ion was performed form this mimetic. The superimposition of the structures is shown in Figure 13 and the PCA results in Figure 14.

The loop H3 is relatively deformed during the dynamics but the 2 loops are still at the correct distance from each other and the PCA on the backbone is not too different. The single loop C (L1) of EC₂ has been synthesised and exhibits a low biological activity. The synthesis of EC is under process; where the addition of the 2 loops is predicted to increase the biological activity compared with loop C (L1) alone.

### C) H3L1L3 Mimetic.

After testing several types of linkages between L1 and L3 to design H3L1L3, it appears that the most stable mimetics are found when L3 is longer than the initial selection, and when L3 is linked both to L1 and H3.

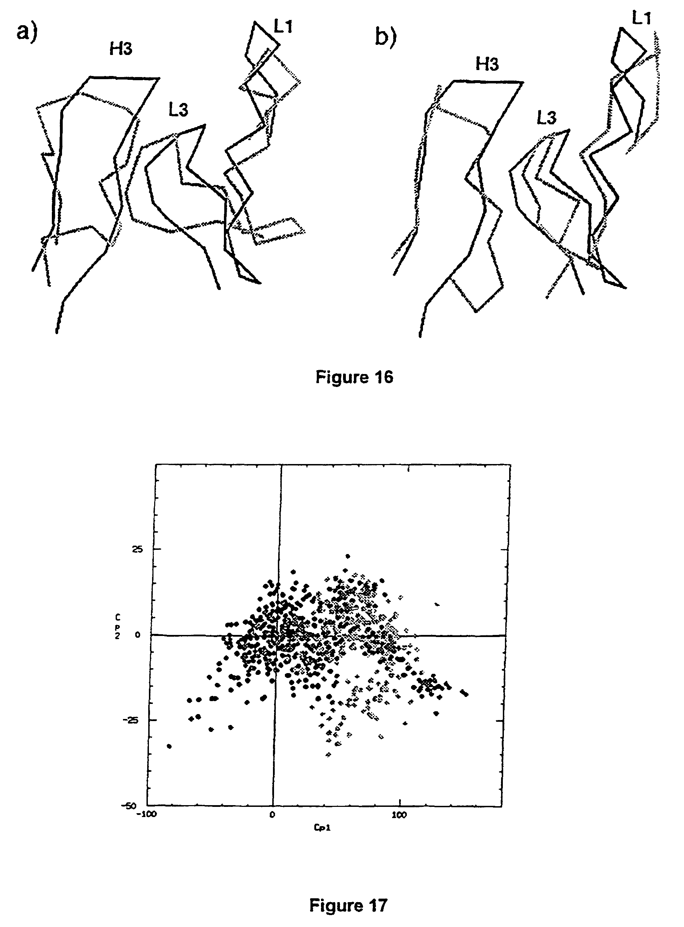

Finally the mimetic E[WFK]2 (Figure 15) or E[WFK]3, where an Orn instead of a Lys was used to link H3 and L1, were selected for synthesis. The MD simulations of these 2 mimetics were carried out and the results are presented on Figures 16 and 17. When Orn is present, the model is more compact than with Lys and has a better RMSD, due to the deformation of L3 with the Lys.

It is clear that it will be difficult to obtain a mimetic exactly in the same conformation as ST40 because of the absence of several loops, but if at least the mimetic H3L1L3 remains stable during the dynamics simulation, as observed for instance with E[WFₖ]3, it is likely to exhibit some stability in solution. The synthesis of E[WFₖ]3 may be difficult, and in this case the link between H3 and L3 may have to be eliminated.

Table 1. The solvent accessible surface areas of the important (red) and less important residues for the 3D model of ST40 was calculated by the SALVOL program developed by R.S. Pearlman *et al*. and implemented in Sybyl. The bold amino acids are those selected to be part of the mimetics.

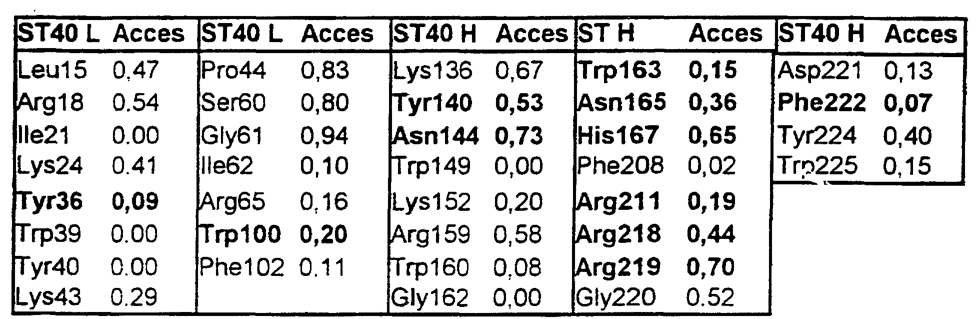

**Table2:**

| Antiviral activity assay on PBMCs infected by HIV isolates. The results are expressed in of inhibition. | | | | | | |
|---|---|---|---|---|---|---|
| **AZT** | **Concentration** | 10 mM | 1 mM | 100 nM | 10 nM | 1 nM |
| | % inhibition | **100 ± 0** | **100 ± 0** | **100 ± 0** | **74 ± 13** | **66 ± 5** |
| **Q4120** | **Concentration** | 5 mg/ml | 500 ng/ml | 250 ng/ml | 50 ng/ml | 25 ng/ml |
| | % inhibition | **96 ± 0** | **56 ± 16** | **41 ± 4** | **8 ± 11** | **-13 ± 6** |
| **ST40** | **Concentration** | 100 mg/ml | 10 mg/ml | 1 mg/ml | 100 ng/ml | 10 ng/ml |
| | % inhibition | **96 ± 0** | **83 ± 3** | **14 ± 11** | **13 ± 1** | **21 ± 11** |
| **9e+08** | **Concentration** | 100 mg/ml | 10 mg/ml | 1 mg/ml | 100 ng/ml | 10 ng/ml |
| | %inhibition | **-5 ± 8** | **13 ± 29** | **-12 ± 11** | **3 ± 32** | **7±4** |
| **S.P** | **Concentration** | 50 mM | 10 mM | 5 mM | 1 mM | 500 nM |
| | %inhibition | **13 ± 8** | **19 ± 22** | **22 ± 15** | **21 ± 3** | **28 ± 4** |
| **MK3** | **Concentration** | 50 mM | 10 mM | 5 mM | 1 mM | 500 nM |
| | % inhibition | **70 ± 3** | **28 ± 12** | **9 ± 8** | **18 ± 5** | **10±10** |
| S.P. = Scrambled Peptide | | | | | | |
| MK3 = Mimetic MK3Tyr-longlSH | | | | | | |

## Claims

1. A method of designing a mimetic which exhibits an activity associated with a parent molecule, the method including:
identifying a plurality of chemical groups of the parent molecule which are associated with activity;
generating a mimetic including the chemical groups associated with activity and a structure which links the chemical groups;
computationally performing a molecular dynamics simulation of the generated mimetic;
determining from the simulation whether the mimetic obeys a predetermined criterion; and
varying the linking structure to produce a second mimetic which better obeys the predetermined criterion.

2. A method according to claim 1 for designing a mimetic of an antibody binding region, the method comprising:
providing the three-dimensional structure of at least the target binding region of the antibody variable domain;
identifying amino acid residues within the region potentially associated with target binding based on experimental data;
computationally determining the respective degree of accessibility of each of the identified amino acid residues within the three-dimensional structure;
selecting a plurality of amino acid residues associated with target-binding activity from among the identified amino acids;
generating a mimetic including the plurality of amino acids, the amino acids groups having within the mimetic a relative geometrical arrangement corresponding to their determined relative geometrical arrangement in the parent antibody,
computationally performing a molecular dynamics simulation of the generated mimetic;
determining from the simulation whether the mimetic obeys a predetermined criterion; and
varying the linking structure to produce a second mimetic which better obeys the predetermined criterion.

3. A method according to claim 2 wherein the amino acid residues which are associated with target binding include at least one first residue which is located on a first loop of the antibody variable domain, and at least one residue which is not located on the first loop of the antibody variable domain; and
designing a mimetic which comprises a first cyclic portion including the first residue, and outside the first cyclic portion the second residue.

4. A method according to claim 3 wherein at least one said second residue is located on a second loop of the antibody variable domain; and
wherein said mimetic which comprises a first cyclic portion including the residue, and outside the first cyclic portion a second cyclic portion comprising at least said second residue.

5. A method according to any of claims 1 to 4 wherein the mimetic is a peptide.

6. A method according to claim 5 wherein the parent molecule is an antibody which binds CD4 and the mimetic is a peptide which binds CD4.

## Patentansprüche

1. Verfahren zum Entwerfen eines Mimetikums, das mit einem Ausgangsmolekül verbundene Aktivität aufweist, wobei das Verfahren Folgendes umfasst:
Identifizieren mehrerer chemischer Gruppen des Ausgangsmoleküls, die mit der Aktivität verbunden sind;
Bilden eines Mimetikums, das die chemischen Gruppen, die mit Aktivität verbunden sind, und eine Struktur umfasst, welche die chemischen Gruppen verbindet;
Durchführen einer Moleküldynamik-Simulation des gebildeten Mimetikums am Computer;
Bestimmen aus der Simulation, ob das Mimetikum einem vorgegebenen Kriterium folgt;
Variieren der Bindungsstruktur, um ein zweites Mimetikum herzustellen, das dem vorgegebenen Kriterium besser folgt.

2. Verfahren nach Anspruch 1 zum Entwerfen eines Mimetikums einer Antikörperbindungsregion, wobei das Verfahren Folgendes umfasst:
Bereitstellen der dreidimensionalen Struktur zumindest der Zielbindungsregion der variablen Antikörperdomäne;
Identifizieren von Aminosäureresten innerhalb der Region, die möglicherweise mit der Zielbindung zusammenhängen, auf Basis von experimentellen Daten;
Bestimmen des jeweiligen Zugänglichkeitsgrades der einzelnen identifizierten Aminosäurereste innerhalb der dreidimensionalen Struktur am Computer;
Auswählen mehrerer Aminosäurereste, denen Zielbindungsaktivität zugeordnet wird, aus den identifizierten Aminosäuren;
Bilden eines Mimetikums, das die Vielzahl an Aminosäuren umfasst, wobei die Aminosäuregruppen innerhalb des Mimetikums eine relative geometrische Anordnung aufweisen, die ihrer ermittelten relativen geometrischen Anordnung im Ausgangsantikörper entspricht;
Durchführen einer Moleküldynamik-Simulation des gebildeten Mimetikums am Computer;
Bestimmen aus der Simulation, ob das Mimetikum einem vorgegebenen Kriterium folgt;
Variieren der Bindungsstruktur, um ein zweites Mimetikum herzustellen, das dem vorgegebenen Kriterium besser folgt.

3. Verfahren nach Anspruch 2, worin die Aminosäurereste, denen Zielbindung zugeordnet wird, zumindest einen ersten Rest, der sich auf einer ersten Schleife der variablen Antikörperdomäne befindet, und zumindest einen Rest umfassen, der sich nicht auf der ersten Schleife der variablen Antikörperdomäne befindet; und
Entwerfen eines Mimetikums, das einen ersten zyklischen Abschnitt mit dem ersten Rest umfasst und außerhalb des ersten zyklischen Abschnitts den zweiten Rest umfasst.

4. Verfahren nach Anspruch 3, worin sich zumindest der zweite Rest auf einer zweiten Schleife der variablen Antikörperdomäne befindet; und
worin das Mimetikum einen ersten zyklischen Abschnitt mit dem Rest umfasst und außerhalb des ersten zyklischen Abschnitts einen zweiten zyklischen Abschnitt umfasst, der zumindest den zweiten Rest umfasst.

5. Verfahren nach einem der Ansprüche 1 bis 4, worin das Mimetikum ein Peptid ist.

6. Verfahren nach Anspruch 5, worin das Ausgangsmolekül ein Antikörper ist, der CD4 bindet, und das Mimetikum ein Peptid ist, das CD4 bindet.

## Revendications

1. Un procédé de conception d'un mimétique qui présente une activité associée à une molécule parente, le procédé comprenant les étapes consistant à:
identifier une pluralité de groupes chimiques de la molécule parente qui sont associés à l'activité;
produire un mimétique comprenant les groupes chimiques associés à l'activité et une structure qui se lie aux groupes chimiques;
effectuer par ordinateur une simulation de dynamique moléculaire du mimétique produit;
déterminer à partir de la simulation si la mimétique obéit à un critère prédéterminé; et
faire varier la structure de liaison pour produire un second mimétique qui obéit mieux au critère prédéterminés.

2. Un procédé selon la revendication 1 pour concevoir un mimétique d'une zone de liaison d'anticorps, le procédé comprenant les étapes consistant à:
créer la structure tridimensionnelle d'au moins la zone de liaison recherchée du domaine variable d'anticorps;
identifier des résidus d'acides aminés à l'intérieur de la zone potentiellement associée à la liaison recherchée sur la base de données expérimentales;
déterminer par ordinateur le degré respectif d'accessabilité de chacun des résidus d'acides aminés identifiés à l'intérieur de la structure tridimensionnelle;
choisir une pluralité de résidus d'acides aminés associés à l'activité de la liaison recherchée parmi les acides aminés identifiés;
produire un mimétique comprenant la pluralité d'acides aminés, les groupes d'acides aminés ayant à l'intérieur du mimétique une disposition géométrique relative correspondant à leur disposition géométrique relative déterminée dans l'anticorps parent,
effectuer par ordinateur une simulation de dynamique moléculaire du mimétique produit;
déterminer à partir de la simulation si le mimétique obéit à un critère prédéterminé; et
faire varier la structure de liaison pour produire un second mimétique qui obéit mieux au critère prédéterminés.

3. Un procédé selon la revendication 2 dans lequel les résidus d'acides aminés qui sont associés à la liaison recherchée comprennent au moins un premier résidu qui est situé sur une première boucle du domaine variable d'anticorps, et au moins un résidu qui n'est pas situé sur la première boucle du domaine variable d'anticorps; et
concevoir un m imétique qui comporte une première partie cyclique comprenant le premier résidu et, à l'extérieur de la première partie cyclique, le second résidu.

4. Un procédé selon la revendication 3, dans lequel au moins un desdits seconds résidus est situé sur une seconde boucle du domaine variable d'anticorps; et
dans lequel ledit mimétique comporte une première partie cyclique comprenant le résidu et, à l'extérieur de la première partie cyclique, une seconde partie cyclique comprenant au moins ledit second résidu.

5. Un procédé selon l'une quelconque des revendications 1 à 4, dans lequel le mimétique est un peptide.

6. Un procédé selon la revendication 5, dans lequel la molécule parente est un anticorps qui se lie à CD4 et le mimétique est un peptide qui se lie à CD4.
